# EUROPÄISCHE PATENTSCHRIFT

(11) **EP 2 627 410 B1**
(45) Veröffentlichungstag und Bekanntmachung des Hinweises auf die Patenterteilung: **12.08.2015**
(21) Anmeldenummer: 11764779.2
(22) Anmeldetag: 07.10.2011
(51) Int. Cl.: A61N 5/10

(54) **VERFAHREN ZUR ERSTELLUNG EINER BESTRAHLUNGSPLANUNG**
METHOD FOR SETTING UP A RADIATION PLANNING
PROCÉDÉ D'ÉTABLISSEMENT D'UNE PLANIFICATION D'IRRADIATION

(30) Priorität: 12.10.2010 DE 102010048233
(43) Veröffentlichungstag der Anmeldung: 21.08.2013
(73) Patentinhaber: GSI Helmholtzzentrum für Schwerionenforschung GmbH, 64291 Darmstadt (DE)
(72) Erfinder: BERT, Christoph, 63741 Aschaffenburg (DE); LÜCHTENBORG, Robert, 64827 Darmstadt (DE)
(86) Internationale Anmeldenummer: PCT/EP2011/067553
(87) Internationale Veröffentlichungsnummer: WO 2012/049085

(56) Entgegenhaltungen:
- WO-A2-2008/106522
- US-A1- 2010 177 871

## Beschreibung

Die Erfindung betrifft ein Verfahren zur Erstellung einer Bestrahlungsplanung für eine Strahlungserzeugungsvorrichtung. Weiterhin betrifft die Erfindung ein Verfahren zur Applizierung einer ortsaufgelösten Strahlendosis in zumindest einer Bestrahlungsposition unter Verwendung zumindest einer Strahlungserzeugungsvorrichtung. Darüber hinaus betrifft die Erfindung eine Vorrichtung zur Erstellung einer Bestrahlungsplanung, eine Vorrichtung zur Ansteuerung zumindest einer Strahlungserzeugungsvorrichtung sowie eine Strahlungserzeugungsvorrichtung, die zumindest eine Vorrichtung zur Ansteuerung der Strahlungserzeugungsvorrichtung aufweist.

Heutzutage kommt es in unterschiedlichsten Bereichen der Technik zu einer Bestrahlung von Gegenständen. Je nach konkretem Einsatzerfordernis werden dabei unterschiedlichste Bestrahlungsverfahren sowie verschiedene Strahlungsarten (beispielsweise Photonenstrahlung, Teilchenstrahlung usw.) verwendet.

In manchen Gebieten der Technik ist es beispielsweise erforderlich, Gegenstände flächig bzw. räumlich zu bestrahlen, wobei die Strahlung möglichst gleichmäßig einwirken sollte. Diese Forderung liegt beispielsweise dann vor, wenn Materialien gehärtet oder in sonstiger Weise bezüglich ihrer Materialeigenschaften verändert werden sollen. Auch im Bereich der Lebensmitteltechnik ist es zwischenzeitlich üblich geworden, Lebensmittel unter Verwendung von bestimmten Strahlungsarten haltbar zu machen.

In anderen Gebieten der Technik ist es dagegen erforderlich, nur bestimmte Bereiche des zu bestrahlenden Gegenstands mit einer bestimmten, typischerweise besonders hohen Dosis zu bestrahlen, während übrige Teile des Gegenstands nicht, bzw. so wenig wie möglich bestrahlt werden sollten. Ein Beispiel hierfür ist die Strukturierung von Mikroprozessoren oder sonstigen Mikrostrukturen bzw. Nanostrukturen unter Verwendung von elektromagnetischer Strahlung (zum Teil bis in den Röntgenbereich hinein und darüber hinaus) sowie bildgebender Masken.

Eine örtlich unterschiedliche Dosisverteilung erfolgt dabei nicht notwendigerweise nur zweidimensional strukturiert, sondern in einigen Technikbereichen auch in allen drei Raumrichtungen strukturiert. Bei einer derartigen, dreidimensionalen Strukturierung der einwirkenden Strahlung ist es beispielsweise möglich, einen Volumenbereich, der sich im Inneren eines zu bestrahlenden Körpers befindet, direkt und unmittelbar zu bestrahlen, ohne den Körper (insbesondere dessen Außenhülle) beschädigen bzw. öffnen zu müssen.

Dabei tritt manchmal das Problem auf, dass der zu bestrahlende Körper (bzw. ein in dessen Inneren liegender Volumenbereich) nicht nur statisch/unbewegt vorliegt. Vielmehr kann es vorkommen, dass sich der Zielkörper bzw. Teile des Zielkörpers (insbesondere der zu bestrahlende Volumenbereich) bewegen. Eine Bewegung kann nicht nur translatorisch relativ zu einem äußeren Koordinatensystem erfolgen, sondern auch in Form einer Verschiebung verschiedener Bereiche des zu bestrahlenden Körpers relativ zueinander (einschließlich Verdrehungen, Verformungen, Stauchungen und/oder Dehnungen) erfolgen.

Um derartige, (in sich) bewegte Körper bestrahlen zu können, werden sogenannte 4D-Bestrahlungsverfahren (vierdimensionale Bestrahlungsverfahren) verwendet. Dabei handelt es sich schlussendlich um dreidimensionale Bestrahlungsverfahren, die eine zeitliche Variation aufweisen (mit der Zeit als der vierten Dimension). Beispiele für derartige Materialbearbeitungsverfahren gibt es im Bereich der Materialwissenschaften bei der Herstellung hochintegrierter Bauteile (insbesondere Mikroprozessoren und Speicherchips) sowie bei der Herstellung von mikrostrukturierten und nanostrukturierten Mechaniken.

Ein weiteres Gebiet der Technik, bei dem dreidimensionale und vierdimensionale Bestrahlungsverfahren eingesetzt werden, liegt im Bereich der Medizintechnik. Auch hier ist es in der Regel erforderlich, bestimmte Volumenbereiche innerhalb eines Körpers, wie beispielsweise einen Tumor, mit einer möglichst hohen Dosis zu beaufschlagen. Das umliegende Gewebe soll dabei möglichst wenig bzw. vorzugsweise im Wesentlichen überhaupt nicht mit einer Dosis belastet werden. Dies gilt in besonderem Maße, wenn es sich bei dem umgebenden Gewebe um ein sogenanntes kritisches Gewebe handelt, wie beispielsweise um ein empfindliches Organ (fachsprachlich als OAR für "Organ at Risk" bezeichnet). Hierbei kann es sich beispielsweise um das Rückenmark, um Blutgefäße oder Nervenknoten handeln.

Insbesondere im Bereich der Medizintechnik sollten die angewendeten Verfahren aus nachvollziehbaren Gründen möglichst präzise und fehlerfrei arbeiten. Ein weiteres Problem im Bereich der Medizintechnik liegt darin, dass aufgrund unterschiedlicher Gegebenheiten (z. Bsp. unterschiedlicher Körperbau der Patienten, unterschiedliche Lage, unterschiedliche Größe, unterschiedliche Beschaffenheit der Tumore) eigentlich immer unterschiedliche Ausgangsbedingungen vorliegen. Das heißt, dass die Bestrahlung (Behandlung) des Patienten jeweils individuell erfolgen muss. Diese individuellen Eigenschaften werden nach dem derzeitigen Stand der Technik in aller Regel unter Verwendung sogenannter Bestrahlungsplanungen berücksichtigt. Hierbei wird eine von einem Arzt vorgeschriebene Dosisverteilung für die unterschiedlichen Gewebebereiche in "maschinenlesbare Parameter" umgerechnet. Das heißt, es wird ein Parametersatz berechnet, der besagt, auf welche Weise der Patient mit dem Teilchenstrahl beaufschlagt werden muss, um die vom Arzt verschriebene Dosisverteilung bestmöglich zu erhalten. Hierbei werden beispielsweise Strahllage, Strahlenergie, Strahleintrittsrichtung, zeitliche Führung des Teilchenstrahls (Scanbewegung), Teilchenart und dergleichen ermittelt. Bei der Erstellung der Bestrahlungsplanung ist eine große Anzahl nicht-linearer und komplexer Zusammenhänge zu berücksichtigen. So ist z. Bsp. bei geladenen Teilchen der (an sich unerwünschte) Dosiseintrag in das hinter dem Bragg-Peak, insbesondere jedoch in das vor dem Bragg-Peak liegende Gewebe zu berücksichtigen. Weiterhin ist die sogenannte relative biologische Wirksamkeit (RBW) rechnerisch zu berücksichtigen. Die Wirkung der Strahlung auf das Gewebe hängt nämlich von unterschiedlichen Parametern, wie der Strahlungsart, der Teilchenart, der Teilchenenergie und der Art des bestrahlen Gewebes selbst ab. Darüber hinaus sind Effekte durch Sekundärstrahlung zu berücksichtigen.

Die bereits vorhandene Komplexität wird nochmals vergrößert, wenn eine Bewegung des zu bestrahlenden Patienten bzw. des zu bestrahlenden Zielvolumenbereichs auftritt. Um hier überhaupt noch eine präzise Behandlung durchführen zu können, sind spezielle Verfahren erforderlich. Derzeit werden zur Lösung des Problems oftmals zwei spezielle Verfahren eingesetzt, nämlich sogenannte Gating-Verfahren sowie sogenannte Tracking-Verfahren. Bei Gating-Verfahren wird die Bestrahlungsplanung auf einen bestimmten Bewegungszustand des Patienten hin optimiert. Befindet sich der Patient in eben dieser Bewegungsphase, so erfolgt ein geeigneter Strahlungseintrag. Befindet sich der Patient jedoch in einer anderen Bewegungsphase, so würde das Gewebe des Patienten "völlig falsch" und erratisch bestrahlt. Um eine derartige, in aller Regel nicht akzeptable Fehlbestrahlung zu vermeiden, erfolgt zu Zeiten, in denen sich der Patient in einer außerhalb eines bestimmten Bewegungsfensters liegenden Bewegungsphase befindet, keine Bestrahlung. Es ist leicht einsichtig, dass dies in der Regel zu einer signifikanten Verlängerung der Bestrahlungsdauer führt. Dies ist nicht nur für den Patienten unangenehm (da er länger behandelt werden muss), sondern führt insbesondere auch zu einer deutlichen Kostensteigerung, was ebenfalls unerwünscht ist.

Bei Tracking-Verfahren wird der Lösungsansatz verfolgt, dass die Bewegung des Patienten kontinuierlich verfolgt wird. Dabei wird die Bewegung des Patienten durch eine geeignete Nachsteuerung des Teilchenstrahls ausgeglichen. Eine Nachsteuerung in transversaler Richtung kann durch Ablenkmagnete erfolgen. Eine Nachsteuerung in longitudinaler Richtung kann beispielsweise durch Energiemodulatoren erfolgen. Solche Energiemodulatoren bestehen beispielsweise aus einem Doppelkeil-Paar, dessen relative Position zueinander, sowie zum Teilchenstrahl veränderlich ist. Je nach Stellung der Keile läuft der durch das Doppelkeil-Paar hindurch tretende Teilchenstrahl eine unterschiedliche Wegstrecke durch das (energieabsorbierende) Material der Keile hindurch. Dementsprechend stark wird die Energie der Teilchen gedämpft. Auf diese Weise kann die Energie der Teilchen, und damit die Lage des Bragg-Peaks, innerhalb gewisser Grenzen eingestellt werden. Die nach wie vor erforderliche Bestrahlungsplanung erfolgt bei Tracking-Verfahren dadurch, dass eine Bestrahlungsplanung für eine bestimmte Referenz-Bewegungsphase erstellt wird. Die Abweichungen zwischen aktueller Bewegungsphase und Referenz-Bewegungsphase werden durch die beschriebenen Nachführungen des Teilchenstrahls ausgeglichen. Die für die Nachführung des Teilchenstrahls erforderlichen Parameter sind dabei üblicherweise Teil der Bestrahlungsplan-Optimierung. Mithilfe derartiger Tracking-Verfahren ist es möglich, dass die im aktuellen Zielraster-Voxel einzubringende Dosis (trotz der gegebenenfalls veränderten Position) tatsächlich dort eingebracht wird. Ein Problem stellt jedoch das umliegende Gewebe (insbesondere das in Strahlrichtung proximal ("luv-seitig") des Zielraster-Voxels befindliche Gewebe) dar. Wird zum Beispiel durch eine Bewegung Gewebe gestaucht oder gedehnt, so kommt es üblicherweise in den betreffenden Bereichen zu einem gegenüber dem Referenz-Plan deutlich abweichenden biologisch wirksamen Dosiseintrag. Kommen auch Rotationen des zu bestrahlenden Gewebes hinzu, so kann es auch vorkommen, dass der Teilchenstrahl durch unterschiedliche Gewebebereiche verläuft, als dies im Referenz-Plan der Fall ist, und dadurch eine Dosis in Bereichen deponiert wird, die im Referenz-Plan nicht belastet werden (und umgekehrt). Der tatsächlich erfolgende Dosiseintrag (auch außerhalb des Ziel-Raster-Voxels) kann zwar während der eigentlichen Bestrahlung gemessen und berechnet werden, jedoch ist dieser tatsächliche Dosiseintrag aufgrund der vor der Bestrahlung unbekannten Korrelation zwischen Bewegungsphase und aktuell bestrahltem Zielraster-Voxel nicht vorhersagbar. In der Regel können hier bestenfalls unter Verwendung statistischer Modelle angenäherte Voraussagen gemacht werden. Damit ist es aber auch nicht möglich, die kumulative Gesamtdosis (exakt) vorherzusagen. Die resultierende Bestrahlungsplanung weist somit entsprechende Defizite auf. Dies kann zu einer Verschlechterung der Bestrahlungsqualität, insbesondere durch potentielle Unterdosierungen im Tumorbereich und/oder durch potentielle Überdosierungen im umliegenden Gewebe führen.

In der Offenlegungsschrift WO 2008/106522 A2 wird ein Computerimplementiertes Verfahren zur Optimierung der Bestrahlungsplanung für eine Strahlentherapievorrichtung beschrieben, welche mit einem sich konisch erweiternden Bestrahlungs-Strahl arbeitet, eine Mehrzahl unabhängig voneinander ansteuerbarer Dämpfungselemente für unterschiedliche Strahlrichtungen aufweist, und derart ausgebildet ist, dass sie die Wirkungen einer Patientenbewegung berücksichtigen kann. Das Verfahren umfasst eine Wahrscheinlichkeitsverteilungsfunktionen, die die Patientenbewegung quantitativ ausdrückt, die Identifizierung einer vorgeschriebenen Gesamtdosis in unterschiedlichen Teilen des zu behandelnden Gebiets, die Zuweisung eines Referenzwerts für jeden Teilstrahlbereich unter Verwendung einer iterativen Funktion, die Berechnung einer tatsächlichen Gesamtdosis in den unterschiedlichen Teilen des zu bestrahlenden Gebiets, welche innerhalb der zugewiesenen Fluenzwerte liegt, sowie die Berechnung eines Erwartungswert für die Dosis pro Energiefluenz-Einheit basierend auf der tatsächlichen Gesamtdosis und der Wahrscheinlichkeitsverteilungsfunktion.

In der US-Offenlegungsschrift US 2010/0177871 A1 wird ein Verfahren zur Verwendung bei einer Bestrahlungsplanung oder bei einer Therapiesitzung vorgeschlagen, welches die Bestimmung eines kritischen Organtyps für gesundes Gewebe sowie die Bestimmung des Strahlungseintrag in gesundes Gewebe basierend auf dem kritischen Organtyp umfasst. Das Verfahren umfasst weiterhin die Bestimmung eines kritischen Organtyps für das gesunde Gewebe und die Bestimmung einer Randbedingung für das gesunde Gewebe, wobei die Randbedingung basierend auf unterschiedlichen Bewegungstrajektorien und dem kritischen Organtyp ermittelt wird.

Es besteht somit nach wie vor Bedarf an verbesserten Bestrahlungsverfahren und/oder verbesserten Berechnungsverfahren. Insbesondere sollen die Verfahren genauer und/oder wirtschaftlicher werden.

Die Aufgabe der Erfindung besteht somit darin, ein gegenüber dem Stand der Technik verbessertes Verfahren zur Erstellung einer Bestrahlungsplanung für eine Strahlungserzeugungsvorrichtung, ein gegenüber dem Stand der Technik verbessertes Verfahren zur Applizierung einer ortsaufgelösten Strahlendosis in zumindest einer Bestrahlungsposition unter Verwendung zumindest einer Strahlungserzeugungsvorrichtung, eine verbesserte Vorrichtung zur Erstellung einer Bestrahlungsplanung, eine verbesserte Vorrichtung zur Ansteuerung zumindest einer Strahlungserzeugungsvorrichtung und/oder eine verbesserte Strahlungserzeugungsvorrichtung, die zumindest eine Vorrichtung zur Ansteuerung der Strahlungserzeugungsvorrichtung aufweist, vorzuschlagen.

Die Erfindung löst die Aufgabe.

Es wird ein Verfahren zur Erstellung einer Bestrahlungsplanung für eine Strahlungserzeugungsvorrichtung vorgeschlagen, bei dem die Bestrahlungsplanung eine Mehrzahl an Bestrahlungspositionen aufweist, die zumindest teilweise mit zumindest einem zum Zeitpunkt der Applizierung der Bestrahlungsplanung auftreten Rahmenparameter korrelieren, und das derart durchgeführt wird, dass bei der Erstellung der Bestrahlungsplanung für diese Bestrahlungspositionen mit höherer Wahrscheinlichkeit zu erwartende Korrelationen mit dem zumindest einen Rahmenparameter verstärkt berücksichtigt werden. Dabei werden unterschiedliche Bestrahlungspositionen und/oder unterschiedliche Gruppen von Bestrahlungspositionen mit unterschiedlichen Zuständen des Rahmenparameters korreliert. Die Erstellung einer Bestrahlungsplanung ist ein numerisch sehr aufwändiger Prozess. Aus diesem Grund ist es vorteilhaft, wenn man sich bei der Berechnung der Bestrahlungsplanung auf die "wesentlichen Teile" beschränkt. Wesentlich sind dabei insbesondere Teile, welche beispielsweise statistisch gesehen vergleichsweise häufig auftreten, wo starke Schwankungen auftreten (beispielsweise durch nicht-lineare Effekte, besondere Gewebeübergänge - beispielsweise von einem Knochenbereich in einen "Normalgewebebereich" - und dergleichen). Wenn man dementsprechend derartige besonders relevante Bereiche (insbesondere im Vergleich zu anderen Bereichen) mit einer höheren Genauigkeit versieht (beispielsweise durch ein feineres Berechnungsgitter, eine höhere numerische Genauigkeit, verbesserte Berechnungsmethoden und dergleichen), so kann die Gesamtgenauigkeit der Bestrahlungsplanung erhöht werden, ohne dass der numerische Aufwand für die Erstellung derselben (wesentlich) ansteigt. Gegebenenfalls ist es sogar möglich, dass auch bei (im Wesentlichen) gleichbleibender oder sogar höherer Qualität der Bestrahlungsplanung eine Reduktion des Aufwands bei der Erstellung derselben realisiert werden kann. Die "verstärkte" Berücksichtigung" kann dabei in einem weiten Bereich erfolgen. So ist es beispielsweise möglich, dass diese mit einer höheren Genauigkeit des Berechnungsgitters und/oder einer höheren Genauigkeit der Algorithmen und/oder einer höheren Anzahl an Iterationsschritten berechnet wird. Im Extremfall ist auch an eine "binäre" (oder "quasi-binäre") Betonung der entsprechenden Korrelation zu denken. In einem derartigen Fall wird dann eine Berechnung (Erstellung von Ansteuerungsparametern, Dosiseintrag in vom aktuellen Zielpunkt-Voxel abweichende Gewebebereiche und dergleichen) lediglich für die voraussichtliche/für die zu erwartende/für die am wahrscheinlichsten eintretende Korrelation berechnet ("binäre Berechnung") bzw. lediglich für die in der Nähe der voraussichtlichen/der zu erwartenden/der wahrscheinlichsten Korrelationen liegende Korrelation (vorzugsweise einschließlich der zu erwartenden/der voraussichtlichen/der am wahrscheinlichsten eintretenden Korrelation) berechnet ("quasi-binäre Berechnung"). Wenn es sich bei dem Rahmenparameter beispielsweise um einen Bewegungszyklus (beispielsweise um einen Atemzyklus) handelt, so können die jeweiligen Bestrahlungspositionen jeweils mit einer (vorzugsweise "geeigneten") Bewegungsphase des Bewegungsablaufs korreliert werden. Denkbar ist es, dass eine Bestrahlung dann lediglich zu den Zeitpunkten erlaubt wird, zu denen die entsprechende Korrelation tatsächlich (näherungsweise) eintritt (also eine Art "Gating"-Verfahren durchgeführt wird). Gegenüber bisherigen "traditionellen" Gating-Verfahren kann jedoch durch die vorgeschlagene verstärkte Berücksichtigung von mit höherer Wahrscheinlichkeit zu erwartenden Korrelationen die Effizienz des resultierenden Verfahrens zum Teil deutlich gesteigert werden. Darüber hinaus ist es insbesondere möglich, die Genauigkeit gegenüber "klassischen" Tracking-Verfahren zum Teil deutlich zu erhöhen. Dies betrifft insbesondere Volumenbereiche, die außerhalb des aktuell bestrahlten Zielpunkts-Voxels liegen. Besonders deutlich wird die Verbesserung, wenn Stauchungs- und Dehnungseffekte und/oder Gewebeverdrehungen eine größere Rolle spielen. Die Mehrzahl an Bestrahlungspositionen mit jeweils einem (gegebenenfalls auch mehreren) dazu korrelierenden Rahmenparametern kann auch als eine Art Gating-Verfahren mit einer Mehrzahl von Gating-Zeitfenstern gesehen werden. Dabei kann beispielsweise jeder Bewegungsphase (bei einem bewegten, zu bestrahlendem Körper) ein eigens Gating-Fenster zugewiesen werden. In der Summe können sich die einzelnen Gating-Fenster dabei addieren, sodass schlussendlich ein relativ großer Anteil der gesamten Bestrahlungszeit ein Strahlungseintrag in den zu bestrahlenden Körper erfolgen kann. In ersten Versuchen hat sich ergeben, dass hierbei 20 %, 30 %, 40 %, 50 %, 60 %, 70 %, 80 % bzw. 90 % (und mehr) Anteil der Bestrahlungszeit effektiv genutzt werden kann. Es ist im Übrigen möglich, dass die Bestrahlungsplanung derart gerechnet wird, dass innerhalb eines einzelnen Gating-Fensters/beim Auftreten eines bestimmten Rahmenparameterwerts bzw. beim Auftreten eines bestimmten Rahmenparameter-Intervalls zur weiteren Verbesserung der Bestrahlungsplanung ein ("klassisches") Tracking-Verfahren vorgesehen wird, und dementsprechend entsprechende Geräteparameter berechnet und gespeichert werden. Erste Versuche haben jedoch gezeigt, dass (insbesondere bei einer größeren Anzahl an Rahmenparameterwerten/Rahmenparameter-Intervallen/Gating-Fenstern) oftmals auf ein derartiges Tracking verzichtet werden kann (insbesondere bei Vorliegen "regulärer" Betriebsbedingungen), denn die Ungenauigkeiten, die durch das Tracking induziert werden können, können zum Teil die Genauigkeiten, die mit dem vorgeschlagenen Verfahren (bzw. der vorgeschlagenen Bestrahlungsplanung) erzielt werden können, sogar verschlechtern. Ein weiterer Vorteil des vorgeschlagenen Verfahrens ist neben den bereits genannten Vorteilen (insbesondere der erhöhten Genauigkeit und/oder des geringeren numerischen Aufwands) eine Reduktion der zu speichernden bzw. der zu verarbeitenden Datenmengen. Weiterhin ist es vorteilhaft, wenn bei der Erstellung der Bestrahlungsplanung berücksichtigt wird, dass bei der schlussendlich unter Verwendung der Bestrahlungsplanung erfolgenden Bestrahlung eine Variation des Bestrahlungsablaufs derart erfolgen kann, dass der/die tatsächlich auftretende(n) Rahmenparameter und der/die im Rahmen der Erstellung der Bestrahlungsplanung zu einer bestimmten Bestrahlungsposition als korrelierend angenommene(n) Rahmenparameter zumindest im Wesentlichen miteinander übereinstimmen (können) und/oder nachgeführt werden (können).

Im Übrigen ist es auch möglich (und in der Regel auch vorteilhaft), dass bereits im Rahmen der Erstellung der Bestrahlungsplanung bestimmte Rahmenparameterbereiche zumindest eines Rahmenparameters und/oder bestimmte Korrelationen zwischen zumindest einem Rahmenparameter und zumindest einer Bestrahlungsposition zumindest zeitweise und/oder zumindest bereichsweise ausgeschlossen werden. Dieser Ausschluss kann insbesondere dahingehend erfolgen, dass zu einem späteren Zeitpunkt, insbesondere im Rahmen der Applizierung der Bestrahlungsplanung, diese Rahmenparameterbereiche beziehungsweise derartige Korrelationen nicht auftreten beziehungsweise nicht appliziert werden. Beispielsweise ist es möglich, dass bestimmte Atemphasen (beispielsweise solche, in denen ein höherer Dosiseintrag in Risikoorgane zu erwarten ist) ganz oder teilweise (beispielsweise für bestimmte Energieschichten) aus der Bestrahlungsplanung ausgeschlossen werden. Dies kann beispielsweise dadurch erfolgen, dass in einem solchen Fall der Teilchenstrahl "pausiert" wird und/oder das nächste "sinnvolle" Bestrahlungsvolumen angesteuert wird.

Vorteilhaft ist es, wenn bei den Verfahren eine Aufteilung in einzelne Bestrahlungspositionen und/oder eine Aufteilung in Gruppen von Bestrahlungspositionen zumindest teilweise zeitbasiert und/oder zumindest teilweise rahmenparameterabhängig erfolgt. Auch eine zumindest zeitweise Anwendung einer derartigen Aufteilung kommt in Betracht. Eine zeitbasierte Aufteilung ist in aller Regel besonders einfach und schnell zu bewerkstelligen. Demgegenüber kann mit einer rahmenparameterabhängigen Aufteilung gegebenenfalls bereits vorhandenes Vorwissen sinnvoll berücksichtigt werden. Ein Beispiel wäre, wenn im Falle einer zyklischen Bewegung eines zu bestrahlenden Zielvolumenbereichs die Aufteilung in Bestrahlungspositionen bzw. in Gruppen von Bestrahlungspositionen in Abhängigkeit von der Bewegung des Zielvolumenbereichs, insbesondere in Abhängigkeit von einer Bewegungsphase des Zielvolumenbereichs erfolgt. Beispielsweise können Bestrahlungspositionen bzw. Gruppen von Bestrahlungspositionen, die mit Bewegungsphasen korrelieren, in denen sich der Zielvolumenbereich nur langsam bewegt (zum Beispiel eingeatmeter bzw. ausgeatmeter Zustand) relativ groß gewählt werden (insbesondere hinsichtlich ihres durch sie abgedeckten Zeitfensters). Zu Zeitpunkten, in denen sich der Zielvolumenbereich schnell bewegt (zum Beispiel während des Einatmens bzw. während des Ausatmens) können dagegen die Bestrahlungspositionen bzw. die Gruppen von Bestrahlungspositionen relativ klein sein. Dieses "groß-sein" bzw. "klein-sein" kann sich insbesondere auf die Zeitachse beziehen, sodass, insbesondere beim beschriebenen Beispiel, die Ortsauflösung im Wesentlichen konstant bleiben kann.

Sinnvoll kann es auch sein, wenn bei Erstellung der Bestrahlungsplanung eine sich zeitlich ändernde Dosisleistung der von zumindest einer Strahlungserzeugungsvorrichtung erzeugten Strahlung zumindest teilweise berücksichtigt wird und/oder wenn bei der Erstellung der Bestrahlungsplanung eine Sicherheitsmarge berücksichtigt wird. Auch eine zumindest zeitweise Berücksichtigung der sich zeitlich ändernden Dosisleistung kommt in Betracht. Es hat sich gezeigt, dass insbesondere bei nicht-kontinuierlich arbeitenden bzw. bei intermittierend arbeitenden Teilchenbeschleunigern die Anzahl der Teilchen nicht notwendigerweise konstant bleibt (bei einem Synchrotron beispielsweise der zeitabhängige Verlauf des sogenannten Teilchen-Spills). Die Form des Teilchen-Spills kann jedoch von Teilchen-Spill zu Teilchen-Spill gesehen relativ konstant sein. Wird dies bei der Bestrahlungsplanung berücksichtigt, so kann die Qualität der schlussendlich erfolgenden Bestrahlung gegebenenfalls nochmals verbessert werden. Insbesondere dann, wenn bei der Erstellung der Bestrahlungsplanung eine Sicherheitsmarge berücksichtigt wird, so können - in der Praxis nie ganz zu vermeidende - Schwankungen zwischen der angenommenen RahmenparameterAbfolge und der tatsächlichen Rahmenparameter-Abfolge während der eigentlichen Bestrahlung in aller Regel besonders vorteilhaft ausgeglichen werden. Dadurch ist es insbesondere möglich, die Qualität der schlussendlich erfolgenden Bestrahlung zum Teil signifikant zu verbessern.

Obgleich es möglich ist, die Rahmenparameter aufgrund von statistischen oder sonstigen Überlegungen bei der Erstellung der Bestrahlungsplanung zu berücksichtigen, ist es in der Regel sinnvoll, wenn das Verfahren derart durchgeführt wird, dass der zumindest eine Rahmenparameter zumindest teilweise unter Verwendung von vorzugsweise durch Messung gewonnenen Daten in die Bestrahlungsplanung einfließt. Auch eine zumindest zeitweise Verwendung von derartigen Daten kommt in Betracht. Beispielsweise ist es möglich, dass ein Patient unter Verwendung von Diagnosevorrichtungen (beispielsweise bildgebenden Diagnosevorrichtungen, wie Computertomografieverfahren und/oder Kernspinresonanztomografieverfahren) vor der Erstellung der Bestrahlungsplanung untersucht wird. Dabei können beispielsweise auch Daten über ein für den jeweiligen Patienten typisches Bewegungsmuster (beispielsweise ein typischer Atemzyklus) gewonnen werden (4D-Erfassung). Diese Daten fließen dabei sinnvollerweise in die Erstellung der Bestrahlungsplanung ein. Insbesondere kann das hierbei gewonnene zeitliche Verhalten dazu genutzt werden, um eine besonders vorteilhafte Korrelation zwischen Bewegungsphasen und Bestrahlungspunkten zu erzielen. Wird hierbei eine sinnvolle Sicherheitsmarge berücksichtigt, so kann die schlussendlich erfolgende Bestrahlung typischerweise derart nachgeführt werden, dass auf eine schnellere bzw. langsamere Bewegungsabfolge während der späteren Bestrahlung ausgeglichen werden kann.

Besonders vorteilhaft ist es, wenn das Verfahren derart durchgeführt wird, dass die Erstellung der Bestrahlungsplanung zumindest bereichsweise in mehreren Schritten erfolgt. Insbesondere ist es möglich, dass zumindest ein Erstellungsschritt der Bestrahlungsplanung und/oder zumindest ein Erstellungsschritt zumindest einer Teil-Bestrahlungsplanung für zumindest eine Bestrahlungsposition und/oder zumindest ein Optimierungsschritt der Bestrahlungsplanung und/oder zumindest ein Optimierungsschritt zumindest einer Teil-Bestrahlungsplanung für zumindest eine Bestrahlungsposition durchgeführt wird. Auch eine zumindest zeitweise Erstellung der Bestrahlungsplanung in mehreren Schritten kommt in Betracht. Auf diese Weise ist es in der Regel möglich, dass Berechnungszeit eingespart werden kann (insbesondere zu bestimmten kritischen Zeitpunkten), ohne dass hierbei die Qualität der Bestrahlungsplanung (übermäßig) absinken muss. Es hat sich nämlich gezeigt, dass die anfängliche Erstellung der "Anfangs-Bestrahlungsplanung" rechnerisch besonders aufwändig ist. Dieser Schritt sollte vorzugsweise lediglich ein einziges Mal durchgeführt werden. Mehr oder weniger geringe Abweichungen von der anfänglichen erstellten "Anfangs-Bestrahlungsplanung" können dagegen in der Regel durch Optimierungsverfahren erfolgen. Die Optimierung ist jedoch in aller Regel numerisch deutlich weniger aufwändig (beispielsweise dadurch, dass eine geringere Anzahl an Iterationsschritten durchgeführt werden muss), sodass hier Rechenzeit eingespart werden kann. Gegebenenfalls kann auch eine NachOptimierung bei bereits fixiertem Patienten unmittelbar vor der Bestrahlung erfolgen (wenn bereits Daten über das "Tagesverhalten" der Strahlungserzeugungsvorrichtung und/oder der tatsächlichen Rahmenparameterabfolge (beispielsweise eine tagesaktuelle Bewegungsabfolge, wie insbesondere die tagesaktuelle Atembewegung eines Patienten) vorliegen usw.).

Zusätzlich wird vorgeschlagen, ein Verfahren zur Applizierung einer ortsaufgelösten Strahlendosis in zumindest eine Bestrahlungsposition, ausgenommen zu therapeutischen Behandlung des menschlichen oder tierischen Körpers, unter Verwendung zumindest einer Strahlungserzeugungsvorrichtung derart durchzuführen, dass die durch die zumindest eine Strahlungserzeugungsvorrichtung die in die zumindest eine Bestrahlungsposition eingetragene Dosisleistung zumindest teilweise in Korrelation zu zumindest einem zum Zeitpunkt der Applizierung auftretenden Rahmenparameter variiert wird. Das Verfahren zur Applizierung einer ortsaufgelösten Strahlendosis wird dabei derart durchgeführt, dass die Applizierung der ortsaufgelösten Strahlendosis zumindest bereichsweise unter Verwendung einer Bestrahlungsplanung vom vorab beschriebenen Typ erfolgt. Auch eine zumindest zeitweise Variation in Korrelation zu zumindest einem zum Zeitpunkt der Applizierung auftretenden Rahmenparameter ist denkbar. Ferner ist eine zumindest zeitweise und/oder zumindest teilweise Nutzung einer derartigen Bestrahlungsplanung ist denkbar. Als Strahlungserzeugungsvorrichtung kann vorzugsweise eine Strahlerzeugungsvorrichtung, besonders vorzugsweise eine Teilchen-Strahlerzeugungsvorrichtung verwendet werden, da (geladene) Teilchen in der Regel einen mehr oder weniger stark ausgeprägten Bragg-Peak aufweisen, sodass auch eine "Bewegung" des Teilchenstrahls in longitudinaler Richtung möglich wird. Es ist im Übrigen möglich, dass Strahlungserzeugungsvorrichtungen eine zeitlich schwankende Ausgangsintensität aufweisen. Weiterhin hat sich in der Praxis gezeigt, dass sich insbesondere bei biologischen System praktisch immer gewisse Variationen einstellen. So ist zwar beispielsweise der Atemzyklus bei einem einzelnen Menschen vom Zyklus zu Zyklus oftmals relativ ähnlich. Dennoch kann es beispielsweise aufgrund von Temperatureinflüssen, Nervosität und so weiter zu beispielsweise Verkürzungen bzw. Verlängerungen desselben kommen. Beide Effekte führen dazu, dass es bei der Verwendung von Scan-Verfahren zu einer zufälligen Korrelation zwischen der Bestrahlungsposition (Lage des zu bestrahlenden Zielvolumens) und der Bewegungsphase, in der der Strahlungseintrag in diese Bestrahlungsposition erfolgt, kommt. Wie bereits erwähnt ist es dadurch zwar nach wie vor möglich, dass - trotz der Bewegung - die Dosis tatsächlich in die aktuell zu bestrahlende Bestrahlungsposition eingetragen wird. Die an sich ungewollten, aber nicht zu vermeidenden Dosiseinträge in das umliegende Gewebe (insbesondere in Teilchenstrahlrichtung zur Bestrahlungsposition proximal liegende Gewebebereiche) können jedoch nicht (exakt) vorherbestimmt werden. Dies wurde bislang als intrinsisches, nicht zu lösendes Problem angesehen. Insbesondere schlugen einige bereits durchgeführte Versuche, die Atmung des Patienten auf die Eigenschaften der Strahlerzeugungsvorrichtung anzupassen, fehl. Dies gilt insbesondere dann, wenn "weiche" Verfahren, verwendet wurden (beispielsweise Aufforderung an den Patienten, auf eine bestimmte Weise zu atmen). Zum Teil wurden in diesem Zusammenhang auch bereits "rigide" Verfahren wie eine Zwangsbeatmung unter Narkose durchgeführt, die jedoch entsprechend aufwändig und belastend für den Patienten sind und dementsprechend in der Kritik stehen. Die Erfinder schlagen daher mit anderen Worten vor, dass bei Durchführung der Bestrahlung die Anpassung an (zum Teil variierende) Rahmenparameter, die im Laufe der Bestrahlung auftreten (wie beispielsweise die Atmung eines Patienten), dadurch erfolgt, dass die Strahlungserzeugungsvorrichtung (insbesondere deren Dosisleistung oder die Länge von Bestrahlungspausen) nachregelt wird. Dadurch ist es möglich, dass der "nominelle" Zusammenhang zwischen Bewegungsphase und Bestrahlungsposition (zumindest in guter Näherung) aufrecht erhalten bleiben kann, und zwar auch dann, wenn es zu gewissen Schwankungen bei dem Rahmenparameter (wie beispielsweise der Bewegung) kommt. Dadurch, dass der "nominelle" Zusammenhang zwischen Bestrahlungsposition und Rahmenparameter erhalten bleibt (also deren Korrelation), können nunmehr auch beispielsweise Strahlungseinträge außerhalb der aktuellen Bestrahlungsposition "vorhersagend" bei der Bestrahlungsplanung berücksichtigt werden. Hierdurch kann die Genauigkeit der Bestrahlung oftmals deutlich erhöht werden.

Gerade dann, wenn bei der Erstellung der Bestrahlungsplanung vorteilhafte (beispielsweise durch vorherige Messung ermittelte) Annahmen über die während der eigentlichen Bestrahlung auftretenden Rahmenparameter (einschließlich deren zeitliche Entwicklung) getroffen wurden, und vorzugsweise geeignete Sicherheitsmargen bei der Erstellung der Bestrahlungsplanung berücksichtigt wurden, kann eine besonders genaue Bestrahlung erfolgen, bei der insbesondere auch Dosiseinträge in außerhalb der aktuellen Bestrahlungsposition liegende Volumenbereiche - und damit auch die schlussendlich resultierende kumulierte Dosisverteilung - besonders genau vorher bestimmbar sind.

Vorgeschlagen wird insbesondere, dass das Verfahren derart durchgeführt wird, dass der zumindest eine Rahmenparameter eine Bewegung zumindest einer Bestrahlungsposition, insbesondere eine zumindest zeitweise periodisch erfolgende Bewegung zumindest einer Bestrahlungsposition und/oder eine Dosisleistung der zumindest einen Strahlerzeugungsvorrichtung, insbesondere eine zeitlich variierende maximale Dosisleistung der zumindest einen Strahlungserzeugungsvorrichtung und/oder eine zeitliche Variation der von der Strahlungserzeugungsvorrichtung erzeugten Dosisleistung darstellt. Auch eine zumindest teilweise Zuordnung des zumindest einen Rahmenparameters zu einer periodisch erfolgenden Bewegung kommt in Betracht. Erste Versuche haben ergeben, dass die vorgeschlagenen Verfahren (sowohl einzeln, als auch in Kombination) besonders effektiv sind, wenn der Rahmenparameter eine Bewegung zumindest einer Bestrahlungsposition und/oder eine Dosisleistung der Strahlungserzeugungsvorrichtung darstellt. Bei einer Bewegung zumindest einer Bestrahlungsposition (beziehungsweise eines Zielvolumenbereichs) ist es besonders vorteilhaft (insbesondere in Bezug auf eine Verringerung der Anzahl der durchzuführenden Berechnungen), wenn die Bewegung zumindest teilweise zyklisch und/oder periodisch erfolgt. Hier kann eine Aufteilung der Gesamtzyklen beziehungsweise Gesamtperioden in einzelne Teilabschnitte besonders vorteilhaft erfolgen. Bei den Schwankungen der Dosisleistung der zumindest einen Strahlungserzeugungsvorrichtung kann es sich insbesondere um eine zeitlich variierende maximale Dosisleistung der zumindest einen Strahlungserzeugungsvorrichtung handeln. Dies kann beispielsweise die während eines sogenannten Teilchen-Spills bei einem Teilchensynchrotron freigesetzte Teilchenmenge pro Zeiteinheit sein.

Besonders vorteilhaft ist es, wenn das bzw. die Verfahren verwendet werden, wenn es sich bei der Bewegung zumindest einer Bestrahlungsposition zumindest zeitweise und/ um eine translatorische, eine rotatorische, eine sich gegeneinander verschiebende, eine stauchende und/oder eine dehnende Bewegung handelt und/oder es sich um eine dichtemäßige Veränderung handelt. Auch kommt es in Betracht, dass es sich zumindest bereichsweise um derartige Bewegungen und/oder dichtemäßige Veränderungen handelt. Gerade bei derartigen Bewegungen weisen bislang bekannte Verfahren oftmals größere Probleme auf, so dass dank der vorliegenden Erfindung besonders deutliche Verbesserungen möglich werden.

Weiterhin kann es vorteilhaft sein, wenn die zumindest eine Strahlungserzeugungsvorrichtung zumindest teilweise Teilchenstrahlung erzeugt, insbesondere zumindest teilweise Hadronenstrahlung erzeugt, vorzugsweise zumindest teilweise Protonenstrahlung, Helium-Ionenstrahlung, Kohlenstoff-Ionenstrahlung, Neon-Ionenstrahlung, Sauerstoff-Ionenstrahlung, Pionenstrahlung, Mesonenstrahlung und/oder Schwerionenstrahlung erzeugt. Auch kommt die zumindest zeitweise Erzeugung (derartiger) Teilchenstrahlung in Betracht. Teilchenstrahlung, insbesondere die genannten Arten von Teilchenstrahlung, hat sich bereits in der Vergangenheit als besonders effektiv bei der Behandlung von Tumoren erwiesen. Dies liegt insbesondere am besonders ausgeprägten Bragg-Peak von Teilchenstrahlung, insbesondere der genannten Teilchenstrahlung. Aber auch ansonsten hat die vorgeschlagene Teilchenstrahlung eine in der Regel besonders große zerstörerische Wirkung für Tumorzellen, was vorteilhaft ist.

Vorteilhaft ist es weiterhin, wenn die Variation der eingetragenen Dosisleistung zumindest teilweise durch Variation der von der Strahlungserzeugungsvorrichtung erzeugten Dosisleistung und/oder durch zumindest zeitweises Aussetzen des Dosiseintrags erfolgt. Auch kommt eine zumindest zeitweise Variation der erzeugten Dosisleistung bzw. ein zumindest teilweises Aussetzen des Dosiseintrags in Betracht. Die vorgeschlagenen Methoden zur Anpassung der Dosisleistung haben sich als besonders effektiv bzw. als relativ einfach zu realisieren erwiesen.

Vorteilhaft ist es, wenn bei dem Verfahren unter Standardbedingungen eine geringere als die maximal mögliche Dosisleistung eingetragen wird. Ganz allgemein kann eine Anpassung der Dosisleistung durch unterschiedlich starkes "Entfernen" von Teilchen bzw. durch unterschiedlich starke Nichtapplikation von Teilchen relativ einfach technisch realisiert werden. (Beispielsweise kann die Applikation des Stahls kurzzeitig ausgesetzt werden.) Mit der vorgeschlagenen Weiterbildung des Verfahrens wird es, dank des nun vorhandenen Puffers, möglich, den Puffer zu nutzen, um auch eine Variation in Richtung "höhere Dosisleistung" durchzuführen. Dies kann die Flexibilität und/oder die Genauigkeit des Verfahrens nochmals erhöhen. Selbstverständlich sollte die Größe der Sicherheitsmarge nicht zu groß gewählt werden, da dieser die Zeitdauer der Behandlung entsprechend seiner Größe verlängert. Als günstiger Wert hat sich in ersten Versuchen eine Reduktion von 10 % bis 20 % gegenüber der maximalen Dosisleistung erwiesen. Vorgeschlagen wird darüber hinaus, dass zwischen zumindest einer Bestrahlungsposition und zumindest einer Bewegungsphase der zumindest einen Bestrahlungsposition eine Korrelation erfolgt. Versuche haben ergeben, dass speziell durch eine derartige Korrelation besonders große, ansonsten oftmals auftretende Fehler beseitigt, zumindest jedoch deutlich verringert werden können.

Besonders vorteilhaft ist es weiterhin, wenn die Bestrahlung in Form eines Scanverfahrens, insbesondere eines Raster-Scanverfahrens durchgeführt wird. Dies hat sich im Zusammenhang mit dem bzw. den vorgeschlagenen Verfahren als besonders vorteilhaft erwiesen. Möglich sind aber insbesondere auch so genannte Spot-Scanverfahren sowie kontinuierliche Scanverfahren.

Im Übrigen ist es möglich, dass im Rahmen des Verfahrens zur Applikation einer Strahlungsdosis eine Art temporäre Notabschaltung vorgesehen wird. Wenn beispielsweise der Patient eine "irreguläre" Bewegung durchführt, kann dieser dann vor einer Fehlbestrahlung geschützt werden. Beispielsweise kann bei der Therapie eines Lungentumors eines Patienten die Atmung (z. Bsp. die Bewegung des Brustkorbs oder dergleichen) überwacht werden. Hustet der Patient, so kann der Teilchenstrahl (kurzzeitig) abgeschaltet werden. Dies ist insbesondere deshalb vorteilhaft, da für derartige irreguläre Bewegungen (beispielsweise besonders schnelle Bewegungen) üblicherweise kein sinnvoller Bestrahlungsplan erstellt werden kann. Auch ist es praktisch nicht möglich, für irreguläre Bewegungen eine sinnvolle Korrelation zwischen Rahmenparameter und Bestrahlungsposition herzustellen. Nach einer derartigen, kurzzeitigen Bestrahlungsunterbrechung ist es in der Regel sinnvoll, die Bestrahlung solange ruhen zu lassen, bis die aktuelle Atemphase wieder der vor der Unterbrechung erreichten entspricht. Zusätzlich oder alternativ kann eine derartige, zeitweise Bestrahlungsunterbrechung auch verwendet werden, wenn zumindest einer der Rahmenparameter (beispielsweise die Atembewegung eines Patienten) Werte einnimmt, die in der Bestrahlungsplanung nicht vorgesehen sind. Ein derartiger Fall kann auftreten, wenn die entsprechenden Werte bei einer im Rahmen der Erstellung der Bestrahlungsplanung stattfindenden Messung nicht auftraten. So ist es nicht unüblich, dass beispielsweise ein Patient während der eigentlichen Behandlung zumindest zeitweise mit größerer Amplitude ("besonders tiefe Atemzüge") atmet, als er dies bei der Voruntersuchung im Rahmen der Erstellung der Bestrahlungsplanung tat. Im umgekehrten Fall, in dem die Atemtrajektorie bestimmte Bewegungsphasen gar nicht erreicht (beziehungsweise ein sonstiger Rahmenparameter bestimmte Werte nicht einnimmt) kann für diese Phasen gegebenenfalls auf eine Art "Notfalltracking" zurückgegriffen werden. Diese Phasen können dann beispielsweise unter Anwendung geeigneter Trackingparameter während einer vorzugsweise benachbarten Bewegungsphase (Rahmenparameterphase) bestrahlt werden.

Weiterhin wird eine Vorrichtung zur Erstellung einer Bestrahlungsplanung vorgeschlagen, die derart ausgebildet und eingerichtet ist, dass sie ein Verfahren gemäß der vorherigen Beschreibung durchführt. Die Vorrichtung zur Erstellung der Bestrahlungsplanung weist dann die bereits vorab beschriebenen Vorteile und Eigenschaften in analoger Weise auf.

Weiterhin wird eine Vorrichtung zur Ansteuerung zumindest einer Strahlungserzeugungsvorrichtung und/oder eine Strahlungserzeugungsvorrichtung mit zumindest einer Vorrichtung zur Ansteuerung der Strahlungserzeugungsvorrichtung vorgeschlagen, die derart ausgebildet und eingerichtet ist, dass die resultierende Strahlungserzeugungsvorrichtung zumindest teilweise ein Verfahren vom vorab beschriebenen Typ durchführt. Auch die Vorrichtung zur Ansteuerung zumindest einer Strahlungserzeugungsvorrichtung und/oder die Strahlungserzeugungsvorrichtung weisen dann die bereits vorab beschriebenen Vorteile und Eigenschaften in analoger Weise auf. Auch eine zumindest zeitweise Verwendung eines solchen Verfahrens ist denkbar.

Auch wenn bislang im Wesentlichen auf die Behandlung eines (menschlichen) Patienten abgestellt wurde, ist es selbstverständlich möglich, die Vorschläge auch auf Tiere, biologische Proben (zum Beispiel Zellkulturen), Patienten-Dummys, Phantome und/oder mechanische Werkstücke anzuwenden.

Im Folgenden wird die Erfindung anhand vorteilhafter Ausführungsbeispiele und unter Bezugnahme auf die beigefügte Zeichnung näher erläutert. Es zeigen:
- Fig. 1:: ein vorgelagertes Verfahren zur Erstellung einer Bestrahlungsplanung in Form eines schematischen Flussdiagramms;
- Fig. 2:: ein Verfahren zur Applizierung einer Bestrahlungsplanung zur Bestrahlung eines beweglichen Zielvolumens in einem schematischen Flußdiagramm;
- Fig. 3:: eine Vorrichtung zur Durchführung einer Bestrahlung in einer schematischen Perspektivansicht;
- Fig. 4:: eine Veranschaulichung eines Verfahrens zur Anpassung der Bestrahlungsgeschwindigkeit durch Variation von Pausen-Zeiten;
- Fig. 5:: eine Veranschaulichung eines Verfahrens zur Anpassung der Bestrahlungsgeschwindigkeit durch Variation der Strahlungsleistung.

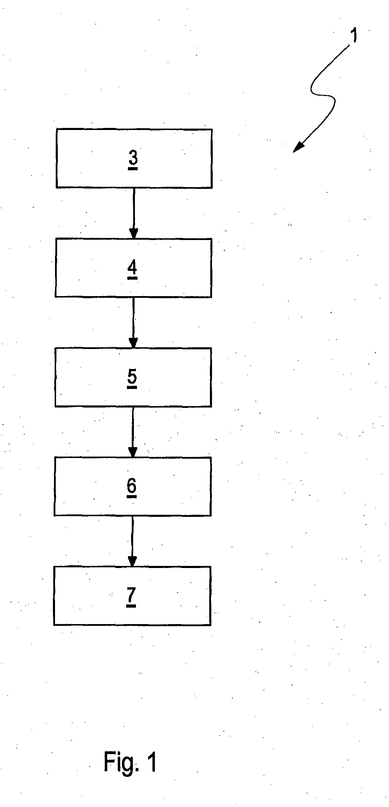

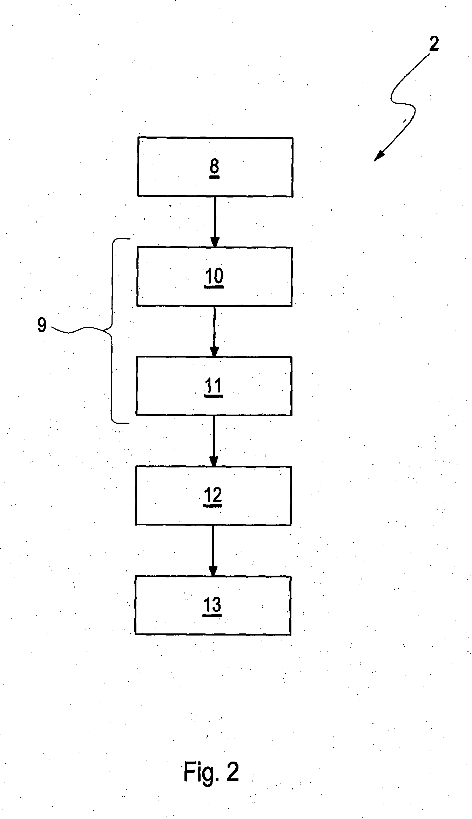

In Fig. 1 ist in schematischer Ansicht ein vorgelagertes Verfahren 1 zur Erstellung einer Bestrahlungsplanung dargestellt. Das vorgelagerte Verfahren 1 erfolgt typischerweise vor der eigentlichen Behandlung (dem eigentlichen Bestrahlungsverfahren 2) und oftmals an einem anderen Ort. Typischerweise erfolgt das vorgelagerte Verfahren 1 mehrere Tage vor dem eigentlichen Bestrahlungsverfahren 2 (siehe Fig. 2).

Beim vorgelagerten Verfahren 1 wird zunächst einmal die genaue Lage und Größe des zu behandelnden Gewebes in einem Untersuchungsschritt 3 bestimmt. Im Rahmen des Untersuchungsschritts 3 werden beispielsweise bildgebende Verfahren, wie insbesondere Computertomografieverfahren und/oder Kernspintomografieverfahren verwendet. Beim vorliegend dargestellten Ausführungsbeispiel erfolgt die Aufnahme der Planungsdaten darüber hinaus zeitaufgelöst (also beispielsweise durch ein 4-D-Computertomografieverfahren beziehungsweise durch ein 4-D-Kernspintomographieverfahren). Vorteilhaft ist es darüber hinaus, wenn gleichzeitig Daten für ein Bewegungssubstitut (wie beispielsweise für einen Dehnungsmessstreifen, welcher um den Brustkorb eines Patienten 22 gelegt ist) aufgenommen werden.

Diese so gewonnenen Daten werden in einem Digitalisierungsschritt 4 in ein für die numerische Erstellung der Bestrahlungsplanung geeignetes Datenformat überführt. Hierbei kann es sich beispielsweise um die Digitalisierung analoger Daten handeln. Auch wenn bereits digitale Daten vorliegen, können - zum Teil aufwändige - Berechnungen zur Umrechnung in ein zur Erstellung der Bestrahlungsplanung geeignetes Datenformat notwendig sein. Beispielsweise kann es für die Erstellung einer Bestrahlungsplanung erforderlich oder sinnvoll sein, Gewebeübergänge zu bestimmen. Hierzu können numerische Verfahren verwendet werden, gegebenenfalls kann aber auch zusätzlich oder alternativ die Eingabe einer Person (gegebenenfalls auch interaktiv) erforderlich sein.

Parallel hierzu (in Fig. 1 nicht dargestellt) kann das im Verfahrensschritt 3 gewonnene Untersuchungsergebnis auch von einem Arzt genutzt werden, um eine Dosis, die während des eigentlichen Bestrahlungsverfahrens 2 (siehe Fig. 2) appliziert werden soll, zu verschreiben.

Die im Digitalisierungsschritt 4 gewonnenen Daten werden unter Berücksichtigung der vom Arzt verschriebenen Dosis im darauffolgenden Verfahrensschritt 5 genutzt, um eine anfängliche Bestrahlungsplanung (Anfangs-Bestrahlungsplanung) zu erstellen. Die Erstellung der Anfangs-Bestrahlungsplanung im Schritt 5 erfolgt beispielsweise unter Verwendung von an sich bekannten Methoden. Beispielsweise kann ein "klassischer" Tracking-Bestrahlungsplan unter Annahme einer Referenz-Position erstellt werden.

Im daran anschließenden Verfahrensschritt 6 wird - basierend auf der in Schritt 5 gestellten Anfangs-Bestrahlungsplanung - ein Satz an "Teil-Bestrahlungsplanungen" erzeugt. Die Art der Unterteilung sowie die Anzahl der "Teil-Bestrahlungsplanungen" basiert dabei auf den im konkreten Fall vorliegenden Rahmenbedingungen. Die einzelnen "Teil-Bestrahlungsplanungen" beziehen sich dabei jeweils auf eine bestimmte Anzahl an Bestrahlungspositionen 33 (gegebenenfalls auch auf eine einzelne Bestrahlungsposition 33). Besonders vorteilhaft ist es dabei, wenn bei der Aufteilung der Anfangs-Bestrahlungsplanung in mehrere Teil-Bestrahlungsplanungen Messwerte, welche im Untersuchungsschritt 3 ermittelt wurden, berücksichtigt werden. Soll beispielsweise ein im Bereich der Lunge eines Patienten 22 liegender Tumor bestrahlt werden, so ist bei der Erstellung des Satzes an Teil-Bestrahlungsplanungen insbesondere die Atembewegung 29 des Patienten zu berücksichtigen. Vorteilhafterweise erfolgt im Falle eines sich bewegenden Tumors die "Aufteilung" der Anfang-Bestrahlungsplanung in Teil-Bestrahlungsplanungen unter Berücksichtigung des Bewegungsmusters des Tumors bzw. des umliegenden Gewebes. So kann nach einer Bewegung des Tumors von beispielsweise jeweils 2 mm eine neue Teil-Bestrahlungsplanung vorgesehen werden. Indirekt kann dadurch die jeweilige Geschwindigkeit des Tumorgewebes in die Erstellung der Teil-Bestrahlungsplanungen eingehen. Die Teil-Bestrahlungsplanungen liegen damit in der Regel zeitlich nicht-äquidistant. Rein beispielhaft bezieht sich ein Teil-Bestrahlungsplan, der mit einem voll-eingeatmeten Zustand des Patienten 22(geringe Tumorgeschwindigkeit) korreliert, auf eine größere Anzahl an Bestrahlungspositionen, als ein Teil-Bestrahlungsplan, der zu einem Bewegungszustand 30 des Patienten 22 korreliert, bei dem dieser aktuell einatmet (große Tumorgeschwindigkeit).

Es ist jedoch auch möglich, dass sich bei manchen Rahmenbedingungen auch eine (zumindest zeitweise) zeitlich äquidistante Beabstandung der Teil-Bestrahlungsplanungen als sinnvoll erweist.

Es ist bevorzugt, wenn in die Erstellung der Teil-Bestrahlungspläne (und damit in den endgültigen Bestrahlungsplan) die Bestrahlungsgeschwindigkeit der Bestrahlungsanlage 14 einfließt. Diese kann beispielsweise hinreichend genau bekannt sein oder - falls schwankend - in hinreichender zeitlicher Nähe zur tatsächlichen Behandlung gemessen werden.

Weiterhin wird die zeitliche Abfolge der einzelnen TeilBestrahlungsplanungen vorliegend so gestaltet, dass die aus den einzelnen Teil-Bestrahlungsplanungen erstellte Gesamt-Bestrahlungsplanung in der vorher berechneten Form insbesondere bei einer gegenüber der maximalen Dosisleistung der Bestrahlungsanlage 14 verringerten Dosisleistung durchgeführt werden kann. Ein typischer Wert ist, dass die Bestrahlungsplanung bei einer 10 - 20 % verringerten Dosisleistung durchgeführt werden kann. Durch diesen "Sicherheitszuschlag" ist es möglich, dass einerseits eine schnellere Atembewegung 29 des Patienten 22 (hervorgerufen beispielsweise durch Nervosität), als auch eine zeitlich variierende Dosisleistung der Bestrahlungsanlage 14 selbst kompensiert werden kann. Selbstverständlich kann eine mit einem derartigen "Sicherheitszuschlag" versehene Bestrahlungsplanung auch dann durchgeführt werden, wenn die Bestrahlungsanlage eine erhöhte Dosisleistung aufweist (beispielsweise können Pausenintervalle 32, in denen der Strahl nicht appliziert wird, in entsprechender Menge und/oder mit einer geeigneten Intervalllänge eingefügt werden). Durch diese Maßnahme kann die in der Bestrahlungsplanung "angenommene" Korrelation zwischen Bewegungsphase 30 des Tumors und der jeweiligen Bestrahlungsposition auch bei der später durchzuführenden Bestrahlung 2 aufrechterhalten werden.

Für die jeweilige Berechnung der Teil-Bestrahlungsplanungen im Schritt 6 können je nach konkretem Erfordernis Optimierungsalgorithmen (ausgehend von der in Schritt 5 erstellten Anfangs-Bestrahlungsplanung) benutzt werden. Bei einer derartigen Verfahrensweise kann üblicherweise ohne wesentlichen Qualitätsverlust eine deutliche Verringerung des numerischen Aufwands realisiert werden. Es kann sich unter bestimmten Rahmenbedingungen jedoch auch als sinnvoll erweisen, jeweils eine vollständig neue Teil-Bestrahlungsplanung zu berechnen (beispielsweise unter Verwendung der im Schritt 5 bei der Erstellung der Anfangs-Bestrahlungsplanung genutzten Verfahren). Der hierdurch erforderliche zusätzliche Zeitbedarf erhöht zwar den Aufwand. Für den Patienten ist dieser erhöhte zeitliche Aufwand jedoch in aller Regel unerheblich, da zwischen dem vorgelagerten Verfahren 1 und dem eigentlichen Bestrahlungsverfahren 2 ohnehin typischerweise mehrere Tage liegen und damit ausreichend Zeit zur Durchführung auch umfangreicher Berechnungen zur Verfügung steht.

Es ist möglich, dass beim Optimierungs-Schritt 6 die Adaptionsparameter des Tracking-Plans (zum Beispiel transversale und/oder longitudinale Verschiebung des Teilchenstrahls) ganz, nur teilweise oder sogar gar nicht berücksichtigt werden. Ein Vorteil einer Nicht-Berücksichtigung beispielsweise der Tiefenmodulation (longitudinale Verschiebung des Teilchenstrahls) kann zum Beispiel den Vorteil haben, dass während der Bestrahlung keine technisch aufwändige Adaption der Teilchenenergie notwendig ist. Aufgrund der bekannten Korrelation zwischen Bestrahlungspunkt (aktuell bestrahltes Ziel-Volumen) und der Bewegungsphase kann die Qualität der schlussendlich applizierten Dosisverteilung dennoch ausreichend hoch sein.

Lediglich der Vollständigkeit halber soll darauf hingewiesen werden, dass bei Verwendung geeigneter Algorithmen ein endgültiger Bestrahlungsplan auch direkt erstellt werden kann (ohne dass ein Verfahrensschritt 6 mit zusätzlichen Optimierungsalgorithmen durchgeführt wird).

Zum Schluss werden die gewonnen Daten (also insbesondere die Anfangs-Bestrahlungsplanung sowie die einzelnen Teil-Bestrahlungsplanungen) gespeichert 7. Hierfür können beliebige, insbesondere an sich bekannte Speichermedien wie beispielsweise Festplatten, CDs, DVDs, Blue-Ray-Discs, Festkörperspeicher (z. Bsp. USB-Sticks) und dergleichen genutzt werden. Zur Erstellung der Bestrahlungsplanung selbst können im Übrigen handelsübliche Computer (PCs, Workstations und dergleichen) verwendet werden. Selbstverständlich ist es auch möglich, dass für die Erstellung von zumindest Teilen der Bestrahlungsplanung auch spezialisierte Hardware genutzt wird.

In Fig. 2 ist - ebenfalls in Form eines schematischen Flussdiagramms - das eigentliche Bestrahlungsverfahren 2 dargestellt.

Zu Beginn des Bestrahlungsverfahrens 2 werden zunächst die vorbereitenden Maßnahmen 8 durchgeführt. So wird der Patient 22 am eigentlichen Behandlungsort 21 immobilisiert und die im vorgelagerten Verfahren 1 errechnete Bestrahlungsplanung wird in die Bestrahlungsvorrichtung 14 eingelesen. Darüber hinaus wird beim vorliegend dargestellten Ausführungsbeispiel für den weiteren Ablauf des Verfahrens nochmals das aktuelle Bewegungsverhalten des zu bestrahlenden Zielvolumenbereichs (beispielsweise bei der Behandlung eines im Bereich der Lunge gelegenen Tumors die Atembewegung 29) und das Extraktionsverhalten des Beschleunigers 14 (Bestrahlungsgeschwindigkeit) nachvermessen. Mit dieser Maßnahme ist es möglich, den tagesaktuellen bzw. behandlungszyklusaktuellen Status der Tumorbewegung einerseits und der Bestrahlungsanlage 14 (insbesondere des Schwerionenbeschleunigers 15) andererseits zu ermitteln. Insbesondere diese beiden Einflussgrößen bestimmen wesentlich die Korrelation zwischen dem jeweiligen Bestrahlungspunkt 33 und der Bewegungsphase 30. In diesem Zusammenhang ist insbesondere an eine gegebenenfalls zeitvariable Dosisleistung über einen einzelnen Teilchenspill hinweg zu denken. Insbesondere bei einem Schwerionenbeschleuniger 15, dessen Verhalten hinreichend genau oder reproduzierbar ist und einem Patienten 22, dessen Tumortrajektorie 29 ausreichend reproduzierbar ist, kann dieser Schritt gegebenenfalls auch entfallen. Zur Erhöhung der Genauigkeit ist es auch optional möglich, die vorgesehene, im vorgelagerten Verfahren 1 berechnete Bestrahlungsplanung zu Testzwecken in ein leeres Cave (beziehungsweise in einen Patienten-Dummy und/oder in ein Phantom) zu schießen. Ein weiterer optionaler Verfahrensschritt besteht darin, dass nochmals eine komplette, zeitaufgelöste Vermessung des Patienten 22 (beispielsweise in Form eines vierdimensionalen Computertomogramms und dergleichen) erfolgt. Hierbei kann gegebenenfalls die Genauigkeit der nachfolgenden Behandlung nochmals erhöht werden. Aufgrund des damit verbundenen Mehraufwands ist ein solches, nochmaliges komplettes Vermessen jedoch üblicherweise auf Ausnahmefälle beschränkt.

Nunmehr wird beim vorliegend dargestellten Ausführungsbeispiel des Bestrahlungsverfahrens 2 im darauf folgenden Verfahrensschritt 9 eine Nachoptimierung der im vorgelagerten Verfahren 1 erstellten Bestrahlungsplanung durchgeführt. Diese Nachoptimierung 9 erfolgt in Form mehrerer Unterschritte, die in Fig. 2 näher beschrieben sind. Die Entscheidung, ob eine Nachoptimierung 9 durchgeführt wird oder nicht, kann insbesondere auch von den vorliegenden Rahmenparametern abhängig gemacht werden (wie insbesondere von der Anatomie des Patienten 22, der aktuellen Atmung 29 des Patienten 22 und/oder dem aktuellen Beschleunigerstatus).

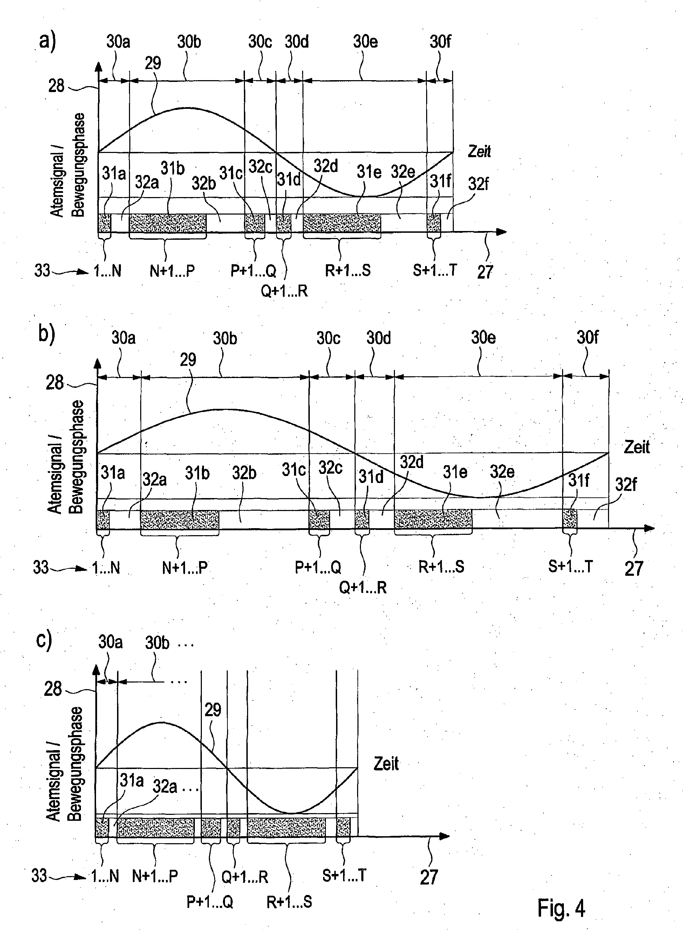

Zunächst einmal werden, basierend auf den während der vorbereitenden Maßnahmen 8 gewonnen Daten und Messergebnisse (insbesondere hinsichtlich des tagesaktuellen Bewegungsmusters 29 des Patienten 22 sowie des tagesaktuellen Verhaltens des Schwerionenbeschleunigers 15 oder sonstiger Teile der Bestrahlungsanlage 14) tagesaktuelle Zeitintervalle 31, 32 für die Bewegungsphasenabfolge 30 ermittelt 10. Auch hier erfolgt die Nach-Optimierung 9 der Bestrahlungsplanung sowie die Aufteilung 10 in einzelne Bewegungsphasen 30 derart, dass der im Folgenden nachzuoptimierende 9 Bestrahlungsplan insbesondere bei einer verminderten Dosisleistung der Bestrahlungsanlage 14 appliziert werden kann. Beim vorliegend dargestellten Bestrahlungsverfahren 2 erfolgt die Anpassung der Bestrahlungsgeschwindigkeit durch die Einfügung von Pausen-Phasen 32 (vergleiche Fig. 4) in der Bestrahlungsplanung, insbesondere von zumindest einer PausenPhase 32 pro Bewegungsintervall 30. Dies kann beispielsweise derart erfolgen, dass die während einer einzelnen Bewegungsphase 30i mit der Zeitdauer t_{B,i} zu bestrahlenden Punkte 33 in einer um eine Sicherheitsmarge t_{safety} verminderten Zeitspanne t_{B,i} - t_{safety} bestrahlt werden können. Während der eigentlichen Bestrahlung kann durch Variation der Pausen-Phase 32 t_{safety} die Bestrahlung derart erfolgen, dass auch bei gegebenenfalls auftretenden Schwankungen die in der Bewegungsphase 30i zu bestrahlenden Rasterpunkte 33 bestrahlt werden können. Dadurch weicht zwar die Zeitdauer t_{B,i} der jeweiligen Bewegungsphase 30i in der Regel von der in der Bestrahlungsplanung angenommenen Dauer t_{B,i} ab. Die (in der Bestrahlungsplanung angenommene) Korrelation zwischen Rasterpunkten 33 und Bewegungsphase 30 kann jedoch (in der Praxis in der Regel mit guter bis hervorragender Näherung) aufrechterhalten werden.

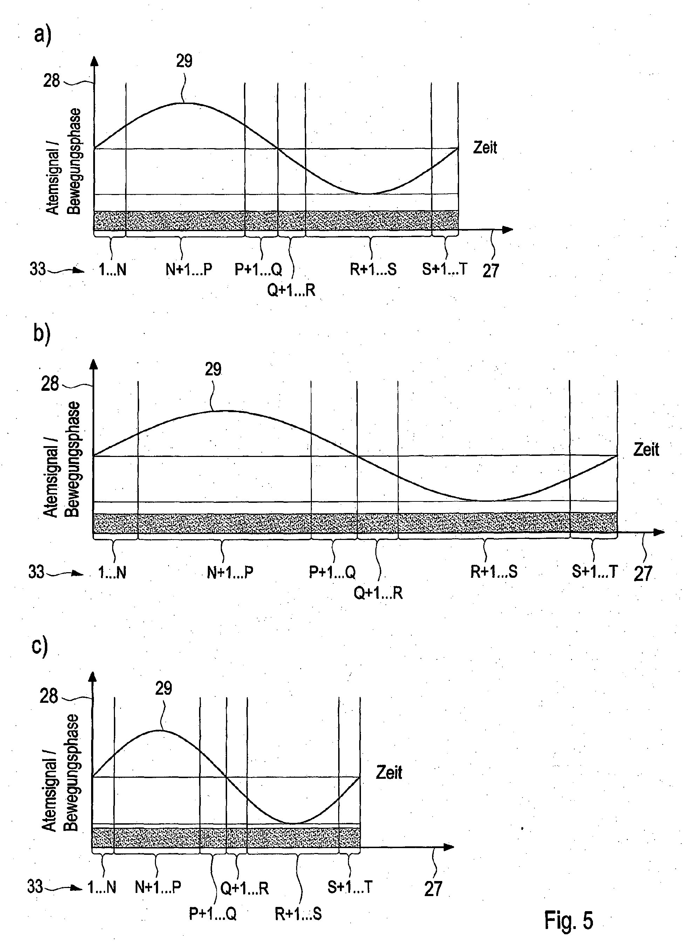

Zusätzlich oder alternativ kann zur Anpassung der Bestrahlungsgeschwindigkeit mit einer modifizierten maximalen Dosisleistung (die unterhalb der maximalen Dosisleistung der Bestrahlungsanlage 14 liegt) geplant werden, falls eine Bestrahlungsanlage 14 verwendet wird, mit der die Dosisleistung während der Behandlung in Echtzeit (als ausreichend schnell) an die Änderungen der Tumorbewegung angepasst werden kann (vergleiche Fig. 5).

Nach Verfahrensschritt 10 steht fest, welcher Rasterpunkt 33 in welcher dazu korrespondierenden Bewegungsphase 30 bestrahlt wird. Darüber hinaus ist gewährleistet, dass pro einzelnem Zeitintervall (Gating-Fenster; Länge t_{B,i} - t_{safety}) nur eine einzelne Bewegungsphase 30 vorliegt (bei Verwendung der Pausen-Phasen-Adaption).

Im Anschluss erfolgt, basierend auf dem im vorgelagerten Verfahren 1 bestimmten vierdimensionalen Bestrahlungsplan (Satz aus einzelnen Teil-Bestrahlungsplänen) die Erstellung einer aktualisierten Bestrahlungsplanung (Schritt 11). Die Erstellung der aktualisierten Bestrahlungsplanung kann dabei in einem ersten Schritt durch Neusortierung der bereits im vorgelagerten Verfahren 1 gewonnenen Daten und einem anschließend durchgeführten Optimierungsschritt für die auf diese Weise neusortierte Bestrahlungsplanung erfolgen. Die Nachoptimierung 9 der aktualisierten Bestrahlungsplanung erfolgt dabei einschließlich der nicht-linearen Effekte, wie beispielsweise des nicht-linearen Einflusses des biologischen Effekts (RBW = relative biologische Wirksamkeit). Der Schritt 11 zur Erstellung der aktualisierten Bestrahlungsplanung ist zwar numerisch anspruchsvoll, jedoch in seinem Umfang noch so weit eingeschränkt, dass es möglich ist, das Nachoptimierungsverfahren 9 bei immobilisierten Patienten 22 durchzuführen. Dies ist insbesondere deshalb möglich, weil im Rahmen des vorgelagerten Verfahrens 1 bereits eine Ausgangsdatenbasis geschaffen wurde, deren Optimierung im Nachoptimierungsschritt 9 im Verhältnis erheblich weniger aufwändig als die Erst-Erstellung während des vorgelagerten Verfahrens 1 ist.

Um die Bestrahlungssicherheit weiter zu erhöhen, ist es möglich, in einem Testbestrahlungsschritt (nicht gesondert eingezeichnet) die im Schritt 9 nachoptimierte Bestrahlungsplanung, basierend auf den realen Patientendaten (einschließlich der aktuell gemessenen Körperbewegung) sowie der Teilchenbeschleunigerdaten in einen Dummy hinein durchzuführen.

Die nunmehr erfolgende, eigentliche therapeutische Bestrahlung 12 beziehungsweise Behandlung wird quasi als gegatete Bestrahlung durchgeführt, wobei jedoch nicht ein einzelnes Gating-Fenster für den gesamten Bewegungszyklus genutzt wird (so wie dies bei "klassischen" Gating-Verfahren der Fall ist), sondern vielmehr eine Vielzahl von Gating-Fenstern 31 a, 31 b, ... genutzt wird, die jeweils für unterschiedliche Bewegungsphasen 30 des Patienten 22 gelten (im Übrigen sollte auch eine gegebenenfalls erfolgende Testbestrahlung vorzugsweise als mehrfach gegatete Bestrahlung durchgeführt werden). Wie oben beschrieben, wird durch die Anpassung der Länge der Gating-Pausen 32 der Erhalt der Korrelation Rasterpunkt 33 - Bewegungsphase 30 sichergestellt. In der Summe ergibt sich somit eine weitgehend kontinuierliche Bestrahlung mit gegenüber "klassischen" Gating-Bestrahlungen üblicherweise verringerten Zeitverlusten. (Bei zusätzlicher und/oder alternativer Verwendung einer Dosisleistungs-Modulation gilt das Entsprechende in Analogie).

Die Bestrahlung des Patienten im Schritt 12 wird selbstverständlich derart durchgeführt, dass sämtliche relevanten Bestrahlungsparameter mitprotokolliert werden, sodass nach der Bestrahlung 12 die tatsächlich deponierte Dosis rekonstruiert werden kann. Dieses Wissen kann in die Bestrahlungsplanung für gegebenenfalls weitere, in der Zukunft (beispielsweise in den nächsten Tagen) erfolgende Bestrahlungszyklen einfließen. Dadurch kann die Güte der durch mehrere Bestrahlungszyklen vorgenommenen Gesamtbestrahlung nochmals erhöht werden.

Die derart mitprotokollierten Daten werden in einem anschließenden Verfahrensschritt 13 abgespeichert (beispielsweise auf CD, DVD, Festplatte, Festkörperspeicher usw.).

Während der eigentlichen Bestrahlung 12 kann es vorkommen, dass der Patient irreguläre Bewegungen durchführt, beispielsweise weil er hustet und/oder besonders tief einatmet. Für eine derartig schnelle beziehungsweise besonders "weite" Bewegung liegen üblicherweise keine Teil-Bestrahlungsplanungen vor, eben weil diese weitgehend erratisch und darüber hinaus eher selten vorliegen. Wird eine derartige, irreguläre Bewegung registriert, so wird die Behandlung durch einen schnellen Abschaltmechanismus kurzzeitig unterbrochen. Dies führt zwar zu einer geringfügigen zeitlichen Verlängerung des Verfahrens, vermeidet jedoch die unerwünschte Deponierung einer Dosis im gesunden Gewebe.

In den Fig. 4 und 5 ist die das Prinzip zur Anpassung der vorab berechneten Bestrahlungsplanung (mit Sicherheitsmarge) an abweichende Rahmenbedingungen zum Zeitpunkt der Applikation der Bestrahlung nochmals verdeutlicht. Dabei wird vorliegend als Beispiel die Atmung 29 eines Patienten 22 verwendet. Das Prinzip ist jedoch auch auf anders geartete Rahmenparameter anwendbar.

Sowohl in Fig. 4, als auch in Fig. 5 ist jeweils längs der Abszisse 27 die fortschreitende Zeit dargestellt. Längs der Ordinate 28 ist jeweils die Atembewegung 29 eines Patienten 22 dargestellt. Die Teilfiguren a (Fig. 4a, 5a) zeigen dabei jeweils die Bewegungsabfolge, die im Rahmen einer Voruntersuchung (beispielsweise Untersuchungsschritt 3 während des vorgelagerten Verfahrens 1) ermittelt wurde. In den Teilfiguren b (Fig. 4b und 5b) ist jeweils die Situation dargestellt, die sich ergibt, wenn es während der eigentlichen Bestrahlung (vergleiche Bestrahlungsverfahren 2) zu einer langsameren Atmung 29 des Patienten 22 kommt. Demgegenüber ist in den Teilfiguren c (Fig. 4c, Fig. 5c) jeweils die Situation dargestellt, wenn der Patient 22 während der eigentlichen Bestrahlung 2 schneller atmet, als während des vorgelagerten Verfahrens 1 angenommen.

In Fig. 4 ist dargestellt, wie sich die Beziehung zwischen Bestrahlungspunkt und Bewegungsphase 30 unter Verwendung von Pausen-Intervallen 32 (im Wesentlichen) aufrecht erhalten lässt. Demgegenüber ist in Fig. 5 dargestellt, wie sich die Korrelation zwischen Bestrahlungspunkten 33 und Bewegungsphase 30 unter Verwendung einer unterschiedlichen Dosisleistung aufrecht erhalten lässt. Die beiden Verfahren sind dabei nicht nur in Alleinstellung, sondern insbesondere auch in Kombination anwendbar. In der Praxis kann die Korrelation sowohl bei Verwendung eines einzelnen Verfahrens, als auch bei Kombination beider Verfahren, in aller Regel in guter bis hervorragender Näherung aufrechterhalten werden.

In Fig. 4a ist zu erkennen, wie sich die Atembewegung 29 in eine Mehrzahl von Bewegungsintervallen 30a, 30b... 30f unterteilen lässt (nach dem Bewegungsintervall 30f beginnt der Atemzyklus 29 mit dem Bewegungsintervall 30a erneut). Bei der Erstellung der Bestrahlungsplanung muss berücksichtigt werden, dass es durchaus wahrscheinlich ist, dass der Patient 22 während der eigentlichen Bestrahlung (bis zu einem gewissen Grad) schneller atmet, als dies während der Voruntersuchung ermittelt wurde. Um hier ausreichend "Spielraum" zu haben, ist bei den einzelnen Bewegungsintervallen 30 jeweils am Anfang eine Bestrahlungsphase 31 vorgesehen, während der eine bestimmte Anzahl an Punkten bestrahlt wird. Beispielsweise werden im ersten Bewegungsintervall 30a die Punkte 33 1 bis N bestrahlt 31a. Jeder Bestrahlungsphase 31 folgt eine Pausenphase 32 nach. Die Länge der jeweiligen Pausenphase 32 bemisst sich vorzugsweise an der zeitlichen Länge der dazu korrespondierenden Bewegungsphase 30 (beispielsweise ein gewisser Prozentsatz wie 10 % oder 20 % hiervon), wobei vorzugsweise eine gewisse Mindestlänge vorgesehen wird. Während der Pausenphase 32 erfolgt keine Bestrahlung. In der vorliegenden Fig. 4 sind die Intervalllängen aus Veranschaulichungsgründen nicht maßstabsgetreu eingezeichnet.

Jeder einzelnen Bewegungsphase 30 (beispielsweise der Bewegungsphase 30a) folgt eine weitere Bewegungsphase 30 (beispielsweise Bewegungsphase 30b) nach, die erneut mit einer Bestrahlungsphase 31 beginnt (beispielsweise 31 b) und schließlich in eine weitere Pausenphase 32 (beispielsweise 32b) übergeht. In diesem nachfolgenden Intervall 30 werden beispielsweise die Bestrahlungspunkte 33 mit den Nummern N + 1 bis P bestrahlt.

Atmet der Patient 22 während der eigentlichen Bestrahlung 2 schneller als ursprünglich "angenommen" (vergleiche Fig. 4a mit 4c) so kann diese schnellere Atembewegung (bis zu einem gewissen Ausmaß) durch eine Verkürzung der Pausenphasen 32 ausgeglichen werden. Durch diese Verkürzung der Pausen-Intervalle 32 ist es möglich, dass die Korrelation zwischen Bestrahlungspunkten 33 und der Bestrahlungsphase 30 trotz der veränderten Atmung 29 des Patienten 22 aufrecht erhalten kann. Konkret werden beispielsweise weiterhin im ersten Bewegungsintervall 30a die Bestrahlungspunkte 33 1 bis N bestrahlt, während im zweiten Bewegungsintervall 30b die Bestrahlungspunkte N + 1 bis P bestrahlt werden. Diese Anpassung des Bestrahlungsfortschritts an die tatsächliche Atembewegung hat den großen Vorteil, dass es möglich ist, den Strahlungseintrag in das um den jeweils aktiven Bestrahlungspunkt 33 herum liegende Gewebe "vorhersagen" zu können.

Natürlich ist es auch möglich, dass der Patient während der eigentlichen Bestrahlung langsamer atmet als im Rahmen der Erstellung 1 der Bestrahlungsplanung angenommen. Um eine derartige Abweichung vom Soll-Wert auszugleichen, werden die jeweiligen Pausen-Phasen 32 geeignet verlängert.

In Fig. 5 ist dargestellt, wie die Anpassung zwischen "angenommener" und "tatsächlicher" Bewegungsgeschwindigkeit durch eine Veränderung der Bestrahlungsintensität vorgenommen werden kann. In diesem Fall kann gegebenenfalls vollständig auf die Verwendung von Pausen-Phasen 32 verzichtet werden. Stattdessen wird die Bestrahlungsintensität bei einer schnelleren Atmung (Fig. 5c) gegenüber der im Rahmen der Bestrahlungsplanung "angenommenen" Atemgeschwindigkeit (Fig. 5a) geeignet erhöht, wohingegen sie bei einer langsameren Atemgeschwindigkeit des Patienten 22 erniedrigt wird (siehe Fig. 5b).

Um eine Anpassung insbesondere auch im Falle einer schnelleren Atmung (Fig. 5c) realisieren können, muss die für die Erstellung der Bestrahlungsplanung verwendete modifizierte maximale Dosisleistung (siehe Fig. 5a) kleiner als die maximale Dosisleistung der verwendeten Bestrahlungsanlage 14 sein. Als geeignete Werte haben sich hierzu 10 bis 20 % Sicherheitsmarge erwiesen. Selbstverständlich setzt die Verwendung des in Fig. 5 veranschaulichten Verfahrens eine hinreichend schnell regelbare Bestrahlungsanlage 14 voraus.

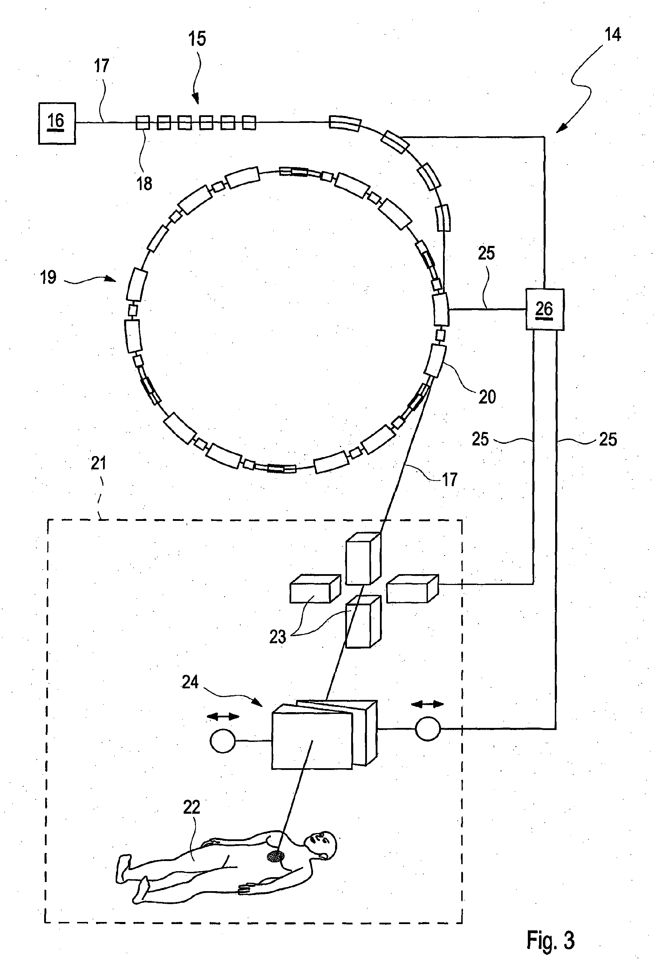

In Fig. 3 ist in einer schematischen, perspektivischen Darstellung eine Bestrahlungsanlage 14 dargestellt, mit der das Bestrahlungsverfahren 2 (gegebenenfalls auch das vorgelagerte Verfahren 1) vorteilhaft durchgeführt werden kann. Die Bestrahlungsanlage 14 ist als an sich bekannter Schwerionenbeschleuniger 15 ausgebildet. Der Schwerionenbeschleuniger 15 weist eine Ionenquelle 16 auf. Die von der Ionenquelle 16 erzeugten Ionen 17 werden zunächst in einem Linearbeschleuniger 18 vorbeschleunigt und in einen Synchrotronring 19 eingeschossen. Im Synchrotronring 19 werden die Ionen 17 weiter auf die erwünschte, hohe Zielenergie beschleunigt. Ist die gewünschte Zielenergie erreicht, werden die im Synchrotronring 19 befindlichen Teilchen über ein Extraktionsseptum 20 extrahiert. Eine typische Zeitdauer für einen Extraktionszyklus (ein sogenannter Teilchenspill) liegt zwischen 2 und 10 Sekunden. Die durch das Extraktionsseptum 20 extrahierten Ionen 17 werden weiter zu einem Behandlungsraum 21, in dem sich der Patient 22 befindet, geführt. Die Applikation der Teilchen (Ionen 17) erfolgt dabei im vorliegend dargestellten Ausführungsbeispiel unter Verwendung eines Scanvorgangs, speziell eines sogenannten Rasterscanvorgangs. Hierzu wird der bleistiftdünne Teilchenstrahl 17 zeilen-, spalten-, und scheibenweise geführt, derart, dass ein erwünschtes dreidimensionales Volumen bestrahlt wird. Je nachdem, wie hoch die im jeweiligen Rasterpunkt einzubringende Einzeldosis ist, verharrt der Teilchenstrahl 17 unterschiedlich lang in der jeweiligen Position.

Die Position des Teilchenstrahls wird dabei in einer lateralen Richtung durch ein Ablenkspulenpaar 23 abgelenkt. In longitudinaler Richtung (Veränderung der Lage des Bragg-Peaks der Ionen 17) erfolgt vorliegend eine Veränderung durch geeignete Ansteuerung eines passiven Energiemodulators 24. Bei diesem werden in an sich bekannter Weise zwei keilförmige Blöcke aus energieabsorbierendem Material (es ist auch eine abweichende Anzahl an Keilen denkbar) derart verfahren, dass der Teilchenstrahl 17 eine unterschiedliche Strecke durch das energieabsorbierende Material zurücklegen muss. Dadurch wird den durch den passiven Energiemodulator 24 hindurch tretenden Ionen 17 unterschiedlich viel Energie entzogen. Möglich ist es aber auch, dass eine (langsamere) Energieanpassung durch eine aktive Anpassung des Synchrotronrings 19 erfolgt. Bei derzeit verfügbaren Synchrotronringen 19 kann eine Energieanpassung von einem Teilchenspill auf den nächsten erfolgen.

Die einzelnen Komponenten der Bestrahlungsanlage 14 stehen über Datenleitungen 25 mit einer Steuereinheit 26 in Verbindung. Die Steuereinheit 26 kann aus einem einzelnen Rechner, oder aus einer größeren Anzahl an Rechnern bestehen. Als Rechner sind nicht nur klassische Computer denkbar, sondern insbesondere auch (teilweise) Einplatinen-Computer und dergleichen. In der Steuereinheit 26 werden die erforderlichen Berechnungen (beispielsweise die im Rahmen des Bestrahlungsverfahrens 2 durchzuführenden Berechnungen) durchgeführt. Weiterhin werden in der Steuereinheit 26 die Steuersignale für die jeweiligen Komponenten 16, 18, 19, 20, 23, 24 erzeugt. Darüber hinaus werden von der Steuereinheit 26 Mess- und Kontrollsignale, die von geeigneten Sensoren bzw. Messgeräten erfasst werden (in Fig. 3 nicht dargestellt), empfangen und geeignet verarbeitet.

## Patentansprüche

1. Verfahren (1, 2) zur Erstellung einer Bestrahlungsplanung für eine Strahlungserzeugungsvorrichtung (14), wobei die Bestrahlungsplanung eine Mehrzahl an Bestrahlungspositionen (33) aufweist, die zumindest teilweise mit zumindest einem zum Zeitpunkt der Applizierung (12) der Bestrahlungsplanung auftretenden Rahmenparameter (29) korrelieren, und wobei bei der Erstellung (1) der Bestrahlungsplanung für diese Bestrahlungspositionen (33) mit höherer Wahrscheinlichkeit zu erwartende Korrelationen mit dem zumindest einen Rahmenparameter (29) verstärkt berücksichtigt werden, **dadurch gekennzeichnet, dass** unterschiedliche Bestrahlungspositionen (33) und/oder unterschiedliche Gruppen von Bestrahlungspositionen (33) mit unterschiedlichen Zuständen des Rahmenparameters (29) korreliert werden.

2. Verfahren (1, 2) nach Anspruch 1, **dadurch gekennzeichnet, dass** die Aufteilung (6) in die Bestrahlungspositionen (33) und/oder die Aufteilung (6) in Gruppen von Bestrahlungspositionen (33) zumindest teilweise zeitbasiert und/oder zumindest teilweise rahmenparameterabhängig erfolgt.

3. Verfahren (1, 2) nach Anspruch 1 oder 2, **dadurch gekennzeichnet, dass** bei der Erstellung (5, 6, 9) der Bestrahlungsplanung eine sich zeitlich ändernde Dosisleistung der von zumindest einer Strahlungserzeugungsvorrichtung (14) erzeugten Strahlung zumindest teilweise berücksichtigt wird und/oder dadurch, dass bei der Erstellung (5, 6, 9) der Bestrahlungsplanung eine Sicherheitsmarge berücksichtigt wird.

4. Verfahren (1, 2) nach einem der vorangehenden Ansprüche, **dadurch gekennzeichnet, dass** der zumindest eine Rahmenparameter (29) zumindest teilweise unter Verwendung von vorzugsweise durch Messung gewonnenen Daten in die Bestrahlungsplanung einfließt (3).

5. Verfahren (1, 2) nach einem der vorangehenden Ansprüche, **dadurch gekennzeichnet, dass** die Erstellung der Bestrahlungsplanung zumindest bereichsweise in mehreren Schritten (5, 6, 9) erfolgt, insbesondere in Form zumindest eines Erstellungsschritts der Bestrahlungsplanung (5) und/oder eines Erstellungsschritts (6) zumindest einer Teil-Bestrahlungsplanung (6) für zumindest eine Bestrahlungsposition (33) und/oder zumindest eines Optimierungsschritts (9) der Bestrahlungsplanung und/oder zumindest eines Optimierungsschritts (9) zumindest einer Teil-Bestrahlungsplanung für zumindest eine Bestrahlungsposition (33).

6. Verfahren (1, 2) zur Applizierung einer ortsaufgelösten Strahlendosis in zumindest einer Bestrahlungsposition (33), ausgenommen zur therapeutischen Behandlung des menschlichen oder tierischen Körpers, unter Verwendung zumindest einer Strahlungserzeugungsvorrichtung (14), vorzugsweise unter Verwendung zumindest einer Teilchenstrahlerzeugungsvorrichtung (15), wobei die zumindest eine Strahlungserzeugungsvorrichtung (14) die in die zumindest eine Bestrahlungsposition (33) eingetragene Dosisleistung zumindest teilweise in Korrelation zu zumindest einem zum Zeitpunkt der Applizierung (12) auftretenden Rahmenparameter (29) variiert wird, **dadurch gekennzeichnet, dass** die Applizierung (12) der ortsaufgelösten Strahlendosis zumindest bereichsweise unter Verwendung einer Bestrahlungsplanung nach einem der Ansprüche 1 bis 5 erfolgt.

7. Verfahren (1, 2) nach einem der vorangehenden Ansprüche, **dadurch gekennzeichnet, dass** der zumindest eine Rahmenparameter eine Bewegung (29) zumindest einer Bestrahlungsposition (33), insbesondere eine zumindest zeitweise periodisch erfolgende Bewegung (29) zumindest einer Bestrahlungsposition (33) und/oder eine Dosisleistung der zumindest einen Strahlungserzeugungsvorrichtung (14), insbesondere eine zeitlich variierende maximale Dosisleistung der zumindest einen Strahlungserzeugungsvorrichtung (14) und/oder eine zeitliche Variation der von der Strahlungserzeugungsvorrichtung (14) erzeugten Dosisleistung darstellt.

8. Verfahren (1, 2) nach Anspruch 7, **dadurch gekennzeichnet, dass** es sich bei der Bewegung zumindest einer Bestrahlungsposition (33) zumindest zeitweise um eine translatorische, eine rotatorische, eine sich gegeneinander verschiebende, eine stauchende und/oder eine dehnende Bewegung handelt und/oder es sich um eine dichtemäßige Veränderung handelt.

9. Verfahren (1, 2) nach einem der vorangehenden Ansprüche, **dadurch gekennzeichnet, dass** die zumindest eine Strahlungserzeugungsvorrichtung (14) zumindest teilweise Teilchenstrahlung erzeugt, insbesondere zumindest teilweise Hadronenstrahlung erzeugt, vorzugsweise zumindest teilweise Protonenstrahlung, Helium-Ionenstrahlung, Kohlenstoff-Ionenstrahlung, Neon-Ionenstrahlung, Sauerstoff-Ionenstrahlung, Pionenstrahlung, Mesonenstrahlung und/oder Schwerionenstrahlung erzeugt.

10. Verfahren (1, 2) nach einem der Ansprüche 6 bis 9, **dadurch gekennzeichnet, dass** die Variation der eingetragenen Dosisleistung zumindest teilweise durch Variation der von der Strahlungserzeugungsvorrichtung (14) erzeugten Dosisleistung und/oder durch zumindest zeitweises Aussetzen des Dosiseintrags erfolgt.

11. Verfahren (1, 2) nach einem der Ansprüche 6 bis 10, bevorzugt nach Anspruch 10, **dadurch gekennzeichnet, dass** unter Standardbedingungen eine geringere als die maximal mögliche Dosisleistung eingetragen wird.

12. Verfahren (1, 2) nach einem der vorangehenden Ansprüche, **dadurch gekennzeichnet, dass** zwischen zumindest einer Bestrahlungsposition (33) und zumindest einer Bewegungsphase (30) der zumindest einen Bestrahlungsposition (33) eine Korrelation erfolgt.

13. Vorrichtung (26) zur Erstellung einer Bestrahlungsplanung, **dadurch gekennzeichnet, dass** diese derart ausgebildet und eingerichtet ist, dass sie ein Verfahren (1, 2) nach einem der Ansprüche 1 bis 12 durchführt.

14. Vorrichtung (26) zur Ansteuerung zumindest einer Strahlungserzeugungsvorrichtung (14) und/oder Strahlungserzeugungsvorrichtung (14), aufweisend zumindest eine Vorrichtung (26) zur Ansteuerung der Strahlungserzeugungsvorrichtung (14), **dadurch gekennzeichnet, dass** diese derart ausgebildet und eingerichtet ist, dass die Strahlungserzeugungsvorrichtung (14) zumindest teilweise ein Verfahren (1, 2) nach einem der Ansprüche 1 bis 13 durchführt.

15. Vorrichtung nach Anspruch 14, **dadurch gekennzeichnet, dass** die zumindest eine Strahlungserzeugungsvorrichtung (14) derart angesteuert wird, dass die in die zumindest eine Bestrahlungsposition (33) eingetragene Dosisleistung zumindest teilweise in Korrelation zu zumindest einem zum Zeitpunkt der Applizierung (12) auftretenden Rahmenparameter (29) variiert wird.

## Claims

1. Method (1, 2) for drafting an irradiation plan for a radiation-generating device (14), wherein said irradiation plan has a plurality of irradiation plans (33) which correlate at least partially with at least one framework parameter (29) appearing at the time of application (12) of the irradiation plan, and wherein during drafting (1) of said irradiation plan for these irradiation plans (33) correlations with the at least one framework parameter (29) that are to be expected with higher probability are taken into greater consideration, **characterized in that** different irradiation plans (33) and/or different groups of irradiation plans (33) are correlated with different states of the framework parameter (29).

2. Method (1, 2) according to claim 1, **characterized in that** the division (6) into the irradiation plans (33) and/or the division (6) into groups of irradiation plans (33) is at least partially time-based and/or at least partially framework-parameter-dependent.

3. Method (1, 2) according to claim 1 or 2, **characterized in that** during drafting (5, 6, 9) of the irradiation plan a dose rate changing over time of the radiation generated by at least one radiation-generating device (14) is at least partially taken into account and/or **in that** during drafting (5, 6, 9) of the irradiation plan a safety margin is taken into account.

4. Method (1, 2) according to one of the preceding claims, **characterized in that** the at least one framework parameter (29) is incorporated (3) into the irradiation plan at least partially using data obtained preferably by measurement.

5. Method (1, 2) according to one of the preceding claims, **characterized in that** the irradiation plan is drafted in several steps (5, 6, 9) at least in some areas, in particular in the form of at least one drafting step of the irradiation plan (5), and/or of a drafting step (6) of at least one partial irradiation plan (6) for at least one irradiation plan (33), and/or of at least one optimization step (9) of the irradiation plan, and/or of at least one optimization step (9) of at least one partial irradiation plan for at least one irradiation plan (33).

6. Method (1, 2) for application of a spatially resolved radiation dose in at least one irradiation plan (33), except for therapeutic treatment of a human or animal body, using at least one radiation-generating device (14), preferably using at least one particle radiation-generating device (15), wherein the dose rate input by the at least one radiation-generating device (14) into the at least one irradiation plan (33) is varied at least partially in correlation with at least one framework parameter (29) appearing at the time of application (12), **characterized in that** the application (12) of the spatially resolved radiation dose is effected at least in some areas using an irradiation plan according to one of claims 1 to 5.

7. Method (1, 2) according to one of the preceding claims, **characterized in that** the at least one framework parameter represents a movement (29) of at least one irradiation plan (33), in particular a movement (29) of at least one irradiation plan (33) taking place at least at some times periodically, and/or a dose rate of the at least one radiation-generating device (14), in particular a maximum dose rate of the at least one radiation-generating device (14) varying over time, and/or a time variation of the dose rate generated by the radiation-generating device (14).

8. Method (1, 2) according to claim 7, **characterized in that** the movement of at least one irradiation plan (33) is at least at some times a translational, rotational, relatively shifting, contracting and/or stretching movement and/or a density-related change.

9. Method (1, 2) according to one of the preceding claims, **characterized in that** the at least one radiation-generating device (14) generates at least partially particle radiation, in particular at least partially hadron radiation, preferably at least partially proton radiation, helium-ion radiation, carbon-ion radiation, neon-ion radiation, oxygen-ion radiation, pion radiation, meson radiation and/or heavy ion radiation.

10. Method (1, 2) according to one of claims 6 to 9, **characterized in that** the variation of the input dose rate is effected at least partially by variation of the dose rate generated by the radiation-generating device (14) and/or by at least partial interruption of the dose input.

11. Method (1, 2) according to one of claims 6 to 10, preferably according to claim 10, **characterized in that** under standard conditions a dose rate lower than the maximum possible one is input.

12. Method (1, 2) according to one of the preceding claims, **characterized in that** a correlation is established between at least one irradiation plan (33) and at least one movement phase (30) of the at least one irradiation plan (33).

13. Device (26) for drafting an irradiation plan, **characterized in that** it is designed and realized such that it performs a method (1, 2) according to one of claims 1 to 12.

14. Device (26) for actuating at least one radiation-generating device (14) and/or radiation-generating device (14) having at least one device (26) for actuating said radiation-generating device (14), **characterized in that** said device (26) is designed and realized such that the radiation-generating device (14) performs at least partially a method (1, 2) according to one of claims 1 to 13.

15. Device according to claim 14, **characterized in that** the at least one radiation-generating device (14) is actuated such that the dose rate input into the at least one irradiation plan (33) is varied at least partially in correlation with at least one framework parameter (29) appearing at the time of application (12).

## Revendications

1. Procédé (1, 2) d'élaboration d'un plan d'irradiation pour un dispositif générateur de rayons (14), sachant que le plan d'irradiation comprend de multiples positions d'irradiation (33), lesquelles sont corrélées au moins partiellement avec au moins un paramètre essentiel (29) apparaissant au moment de l'application (12) du plan d'irradiation, et sachant que lors de l'élaboration (1) du plan d'irradiation pour ces positions d'irradiation (33), les corrélations avec ledit au moins un paramètre essentiel (29) pouvant être attendues avec une plus grande probabilité sont davantage prises en compte, **caractérisé en ce que** différentes positions d'irradiation (33) et/ou différents groupes de positions d'irradiation (33) sont corrélés avec différents états du paramètre essentiel (29).

2. Procédé (1, 2) selon la revendication 1, **caractérisé en ce que** la division (6) en positions d'irradiation (33) et/ou la division (6) en groupes de positions d'irradiation (33) se fait au moins partiellement en fonction du temps et/ou au moins partiellement en fonction des paramètres essentiels.

3. Procédé (1, 2) selon la revendication 1 ou 2, **caractérisé en ce que**, lors de l'élaboration (5, 6, 9) du plan d'irradiation, un débit de dose du rayonnement variant dans le temps et généré par au moins un dispositif générateur de rayons (14) est au moins partiellement pris en compte et/ou **en ce que**, lors de l'élaboration (5, 6, 9) du plan d'irradiation, une marge de sécurité est prise en compte.

4. Procédé (1, 2) selon l'une des revendications précédentes, **caractérisé en ce que** ledit au moins un paramètre essentiel (29) est intégré au plan d'irradiation (3) en utilisant au moins partiellement les données acquises de préférence par mesurage.

5. Procédé (1, 2) selon l'une des revendications précédentes, **caractérisé en ce que** l'élaboration du plan d'irradiation se fait au moins par endroits en plusieurs étapes (5, 6, 9), en particulier sous forme d'au moins une étape d'élaboration du plan d'irradiation (5), et/ou une étape d'élaboration (6) d'au moins un plan partiel d'irradiation (6) pour au moins une position d'irradiation (33), et/ou au moins une étape d'optimisation (9) du plan d'irradiation, et/ou au moins une étape d'optimisation (9) d'au moins un plan partiel d'irradiation pour au moins une position d'irradiation (33).

6. Procédé (1, 2) d'application d'une dose de rayonnement à résolution spatiale dans au moins une position d'irradiation (33), à l'exception d'un traitement thérapeutique du corps humain ou animal, en utilisant au moins un dispositif générateur de rayons (14), de préférence en utilisant au moins un dispositif générateur de faisceaux de particules (15), sachant que ledit au moins un dispositif générateur de rayons (14) fait varier le débit de dose appliqué dans au moins une position d'irradiation (33) au moins partiellement en corrélation avec au moins un paramètre essentiel (29) apparaissant au moment de l'application (12), **caractérisé en ce que** l'application (12) de la dose de rayonnement à résolution spatiale se fait au moins par endroits en utilisant un plan d'irradiation selon l'une des revendications 1 à 5.

7. Procédé (1, 2) selon l'une des revendications précédentes, **caractérisé en ce que** ledit au moins un paramètre essentiel représente un mouvement (29) d'au moins une position d'irradiation (33), en particulier un mouvement (29) de ladite au moins une position d'irradiation (33) s'effectuant périodiquement au moins par moments, et/ou un débit de dose dudit au moins un dispositif générateur de rayons (14), en particulier un débit de dose maximal variant dans le temps dudit au moins un dispositif générateur de rayons (14) et/ou une variation temporelle du débit de la dose généré par le dispositif générateur de rayons (14).

8. Procédé (1, 2) selon la revendication 7, **caractérisé en ce que**, pour le mouvement de ladite au moins une position d'irradiation (33), il s'agit au moins par moments d'un mouvement translatoire, rotatif, se déplaçant par rapport à un autre, de compression et/ou d'expansion et/ou qu'il s'agit d'une modification en fonction de la densité.

9. Procédé (1, 2) selon l'une des revendications précédentes, **caractérisé en ce que** ledit au moins un dispositif générateur de rayons (14) génère au moins partiellement un rayonnement de particules, en particulier au moins partiellement un rayonnement hadronique, de préférence au moins partiellement un rayonnement protonique, d'ions d'hélium, d'ions de carbone, d'ions de néon, d'ions d'oxygène, de pions, de mésons et/ou d'ions lourds.

10. Procédé (1, 2) selon l'une des revendications 6 à 9, **caractérisé en ce que** la variation du débit de dose appliqué se fait au moins partiellement par variation du débit de la dose généré par le dispositif générateur de rayons (14) et/ou par une interruption au moins temporaire de l'application de la dose.

11. Procédé (1, 2) selon l'une des revendications 6 à 10, de préférence selon la revendication 10, **caractérisé en ce que**, dans des conditions standards, le débit de dose appliqué est inférieur au débit maximal possible.

12. Procédé (1, 2) selon l'une des revendications précédentes, **caractérisé en ce qu'**une corrélation est établie entre au moins une position d'irradiation (33) et au moins une phase de mouvement (30) de ladite au moins une position d'irradiation (33).

13. Dispositif (26) pour élaborer un plan d'irradiation, **caractérisé en ce que** ce dernier est conçu et réalisé de telle sorte qu'il exécute un procédé (1, 2) selon l'une des revendications 1 à 12.

14. Dispositif (26) de pilotage d'au moins un dispositif générateur de rayons (14) et/ou dispositif générateur de rayons (14), présentant au moins un dispositif (26) de pilotage du dispositif générateur de rayons (14), **caractérisé en ce que** ledit dispositif (26) est conçu et réalisé de telle sorte que le dispositif générateur de rayons (14) exécute au moins partiellement un procédé (1, 2) selon l'une des revendications 1 à 13.

15. Dispositif selon la revendication 14, **caractérisé en ce que** ledit au moins un dispositif générateur de rayons (14) est piloté de sorte que le débit de dose appliqué dans au moins une position d'irradiation (33) varie au moins partiellement en corrélation avec au moins un paramètre essentiel (29) apparaissant au moment de l'application (12).
